(19) Europäisches Patentamt
European Patent Office
Office européen des brevets

(11) Numéro de publication : **0 099 303**
**B1**

# (12) FASCICULE DE BREVET EUROPÉEN

(45) Date de publication du fascicule du brevet : **19.08.87**

(21) Numéro de dépôt : **83401431.8**

(22) Date de dépôt : **11.07.83**

(51) Int. Cl.⁴ : **C 07 D495/04**, A 61 K 31/435 // (C07D495/04, 335:00, 221:00)

## (54) Nouvelles benzothiopyranopyridinones et leurs sels, leur préparation, leur application comme médicaments et les compositions les renfermant.

(30) Priorité : **14.07.82 GB 8220433**

(43) Date de publication de la demande : **25.01.84 Bulletin 84/04**

(45) *Mention de la délivrance du brevet :* **19.08.87 Bulletin 87/34**

(84) Etats contractants désignés : **AT BE CH DE FR IT LI LU NL SE**

(56) Documents cités :
**EP-A- 0 035 924**
**GB-A- 1 252 131**

(73) Titulaire : **ROUSSEL-UCLAF**
**35, boulevard des Invalides**
**F-75007 Paris (FR)**

(72) Inventeur : **Rowland, David Alun**
**Wyke House Crudwell**
**Malmesbury Wilts (GB)**
Inventeur : **Gillespie, Roger John**
**22 Wentworth Park Freshbrook**
**Swindon Wilts (GB)**
Inventeur : **Ager, Ian Robert**
**Three Willows Gosditch**
**Ashton Keynes Wilts (GB)**
Inventeur : **Clements-Jewery, Stephen**
**56 High Street Blunsdon**
**Swindon Wilts (GB)**
Inventeur : **Gardner, Colin Robert**
**Stoneways Bishopstone Road**
**Bourton, Nr. Swindon (GB)**

(74) Mandataire : **Vieillefosse, Jean-Claude et al**
**Département des Brevets ROUSSEL UCLAF B.P no 9**
**F-93230 Romainville (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**EP 0 099 303 B1**

Jouve, 18, rue St-Denis, 75001 Paris, France

**Description**

La présente invention concerne de nouvelles benzothiopyranopyridinones et leurs sels, ainsi que le procédé de préparation, l'application à titre de médicaments de ces nouveaux produits et les compositions pharmaceutiques les renfermant.

L'invention a pour objet de nouvelles benzothiopyranopyridinones ainsi que leurs sels, caractérisées en ce qu'elles répondent à la formule

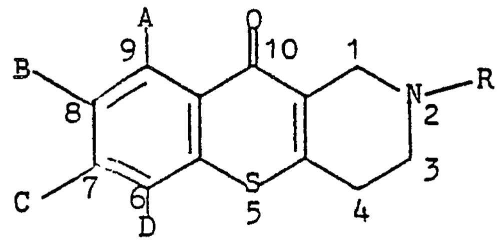

(I)

dans laquelle A, B, C et D, identiques ou différents, représentent un atome d'hydrogène, un radical alcoyle linéaire ou ramifié renfermant de 1 à 6 atomes de carbone, un radical alcoxy linéaire ou ramifié renfermant de 1 à 6 atomes de carbone, un radical nitro, un radical amino, un radical acylamino renfermant de 2 à 7 atomes de carbone ou un atome d'halogène, ou l'un des groupes A et B ou C et D représente avec les atomes de carbone auxquels ils sont liés, un cycle benzénique et l'autre est défini comme ci-dessus,

R représente un atome d'hydrogène ou un radical R' dans lequel R' représente un radical alcoyle, linéaire ou ramifié, renfermant de 1 à 9 atomes de carbone ou alcényle, linéaire ou ramifié, renfermant de 2 à 9 atomes de carbone, éventuellement substitué par un radical hydroxy, un radical cycloalcoyle renfermant de 3 à 9 atomes de carbone, un radical phényle, un radical aralcoyle renfermant de 7 à 9 atomes de carbone ou un groupement $-(CH_2)_n-Z$ dans lequel n est un nombre entier qui varie de 1 à 4 et Z représente un radical cycloalcoyle ou cycloalcényle renfermant de 3 à 9 atomes de carbone, un radical dioxanyl, un radical 2-oxo benzimidazolyl, un radical benzoyl éventuellement substitué par un atome d'halogène, un radical alcoxycarbonyl renfermant de 2 à 7 atomes de carbone, un radical cyano ou un radical carbamoyl.

Dans la formule I et dans ce qui suit, le terme radical alcoyle renfermant de 1 à 6 atomes de carbone désigne par exemple un radical méthyle, éthyle, propyle, isopropyle, butyle ou pentyle ; le terme radical alcoxy renfermant de 1 à 6 atomes de carbone désigne par exemple un radical méthoxy, éthoxy propoxy, isopropoxy, butoxy ou pentoxy ; le terme radical acylamino renfermant de 2 à 7 atomes de carbone désigne par exemple un radical acétamido, propionamido, butyrylamido ; le terme atome d'halogène désigne par exemple un atome de chlore, de fluor ou de brome ; le terme radical alcoyle linéaire ou ramifié, renfermant de 1 à 9 atomes de carbone désigne par exemple un radical méthyle, éthyle, propyle, isopropyle, butyle isobutyle, tert-butyle, pentyle, isopentyle, hexyle, heptyle ou nonyle ; le terme radical alcényle renfermant de 2 à 9 atomes de carbone désigne par exemple un radical vinyle, allyle ; le terme cycloalcoyle ou cycloalcényle renfermant de 3 à 9 atomes de carbone désigne par exemple un radical cyclopropyle, cyclobutyle, cyclopentyle, cyclohexyle, cycloheptyle, cyclobutényl, cyclopentényl ou cyclohexényl ; le terme radical aralcoyle renfermant de 7 à 9 atomes de carbone désigne par exemple un radical benzyle, phénéthyle ou phénylpropyle ; le terme groupement $-(CH_2)_n-Z$ défini ci-dessus, dans lequel Z représente un radical benzoyl éventuellement substitué par un atome d'halogène désigne par exemple un radical phényl-4-oxo butyle, phényl-3-oxopropyle, phényl-2-oxoéthyle ou p-fluorophényl-4-oxobutyle ; le terme radical alcoxycarbonyle renfermant de 2 à 7 atomes de carbone désigne par exemple un radical méthoxycarbonyle, éthoxycarbonyle, propoxycarbonyle ou butoxycarbonyle.

Les produits de formule I présentent un caractère basique ; ils peuvent par conséquent former des sels d'addition avec les acides tels que par exemple les sels formés avec les acides chlorhydrique, bromhydrique, iodhydrique, nitrique, sulfurique, phosphorique, acétique, formique, benzoïque, maléique, fumarique, succinique, tartrique, citrique, oxalique, glyoxylique, aspartique, alcanesulfoniques, tels que l'acide méthane-sulfonique et aryl-sulfoniques, tels que l'acide benzène sulfonique.

Parmi les produits objet de l'invention, on peut citer notamment les dérivés répondant à la formule (I) ci-dessus, ainsi que leurs sels, caractérisés en ce que dans ladite formule (I), A représente un atome d'hydrogène et B représente un atome d'hydrogène, un radical méthyle, méthoxy, un radical nitro ou acétamido ou un atome de chlore ou de fluor ou B forme avec A et les atomes de carbone auxquels ils sont liés, un cycle benzénique, C représente un atome d'hydrogène ou un radical méthoxy et D représente un atome d'hydrogène ou forme avec C et les atomes de carbone auxquels ils sont liés un cycle benzénique, R a la signification déjà indiquée.

Parmi ces derniers, on peut citer les dérivés répondant à la formule (I) ci-dessus, ainsi que leurs sels, caractérisées en ce que R représente un atome d'hydrogène ou un radical R' dans lequel R' représente un radical alcoyle, linéaire ou ramifié, renfermant de 1 à 9 atomes de carbone, un radical benzyle, un radical

cyclohexylméthyle ou un groupement p-fluoro phényl-4-oxo butyl ou éthoxy carbonyléthyle.

Parmi les produits objet de l'invention on retient tout particulièrement ceux caractérisés en ce que A, B, C et D représentent un atome d'hydrogène et notamment

la 1,2,3,4-tétrahydro 2-n-propyl-10H-/1/-benzothiopyrano/3,2-c/ pyridin-10-one et ses sels,

la 1,2,3,4-tétrahydro 2-n-butyl-10H-/1/-benzothiopyrano/3,2-c/ pyridin-10-one et ses sels,

la 1,2,3,4-tétrahydro 2-isopentyl-10H-/1/-benzothiopyrano/3,2-c/pyridin-10-one et ses sels,

la 1,2,3,4-tétrahydro 2-n-pentyl-10H-/1/-benzothiopyrano/3,2-c/ pyridin-10-one et ses sels,

la 1,2,3,4-tétrahydro 2-(4-p-fluorophényl-4-oxobutyl)-10H-/1/-benzothiopyrano /3,2-c/ pyridin-10-one et ses sels,

la 1,2,3,4-tétrahydro 2-cyclohexylméthyl 8-fluoro 10H-/1/-benzothio pyrano /3,2-c/ pyridin-10-one et ses sels,

la 1,2,3,4-tétrahydro 2-cyclopropylméthyl 10H-/1/-benzothiopyrano /3,2-c/ pyridin-10-one et ses sels,

la 1,2,3,4-tétrahydro 2-cyclobutylméthyl 10H-/1/-benzothiopyrano /3,2-c/ pyridin-10-one et ses sels,

la 1,2,3,4-tétrahydro 2-cyclopentylméthyl 10H-/1/-benzothiopyrano /3,2-c/ pyridin-10-one et ses sels et, tout particulièrement,

la 1,2,3,4-tétrahydro 2-cyclohexylméthyl 10H-/1/-benzothiopyrano /3,2-c/ pyridin-10-one et ses sels.

L'invention a également pour objet un procédé de préparation des nouvelles benzothiopyranopyridinones telles que définies par la formule (I) ci-dessus, ainsi que de leurs sels, caractérisé en ce que l'on condense le thiol de formule

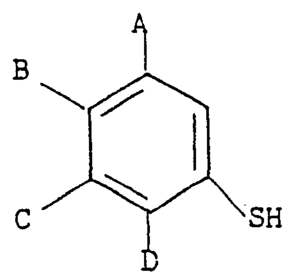

(II)

dans laquelle A, B, C et D ont la signification déjà indiquée avec une 4-pipéridone ou un sel de 4-pipéridone de formule

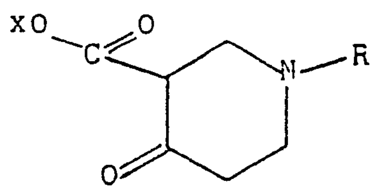

(III)

dans laquelle X représente un radical alcoyle renfermant de 1 à 8 atomes de carbone et R est défini comme précédemment pour obtenir un produit de formule

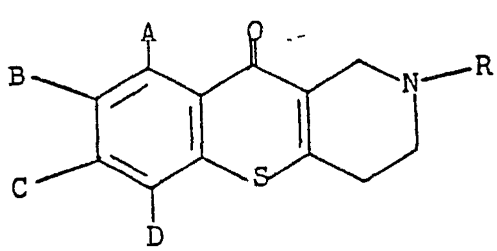

(I)

dans laquelle A, B, C, D et R ont la signification déjà indiquée le cas échéant, isole et, si désiré, salifie ledit produit de formule (I) ainsi obtenu, ou, à condition d'avoir préparé un produit de formule (I) dans laquelle R représente un atome d'hydrogène,

soit on fait réagir ce dernier avec un dérivé halogéné de formule

Hal—R' (IV)

dans laquelle Hal représente un atome de chlore, de brome ou d'iode et R' a la signification indiquée précédemment à l'exception d'un radical phényle, pour obtenir un produit de formule :

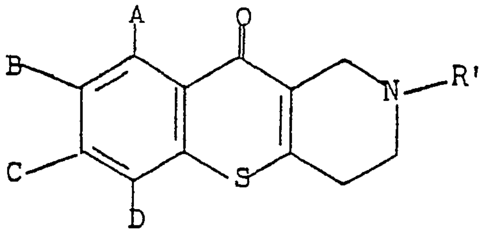

(I')

dans laquelle A, B, C, E et R' ont la signification déjà indiquée et le cas échéant, isole et, si désiré, salifie ledit produit de formule (I') ainsi obtenu,
soit on fait réagir un produit de formule (I) dans laquelle R représente un atome d'hydrogène, avec un produit de formule

$$CH_2 = CH—Z' \qquad (V)$$

dans laquelle Z' représente un radical alcoxycarbonyl, cyano ou carbamoyl, pour obtenir un produit de formule

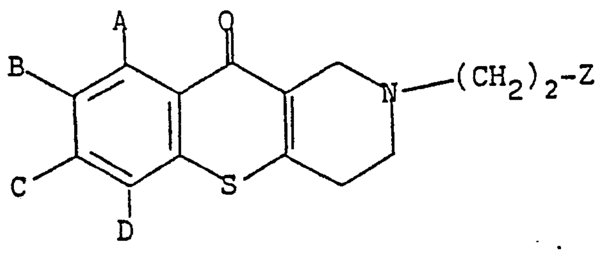

(I")

dans laquelle A, B, C, D et Z' ont la signification déjà indiquée, isole et, si désiré, salifie ledit produit de formule (I") ainsi obtenu.

Dans des conditions préférentielles de mise en œuvre de l'invention, le procédé de préparation ci-dessus décrit est caractérisé en ce que :

la condensation du thiol de formule (II) avec la 4-pipéridone de formule III est effectuée par chauffage pendant plusieurs heures à plusieurs jours, au sein d'un acide fort tel que l'acide polyphosphorique ;

lorsque l'on utilise un sel de 4-pipéridone, il s'agit du chlorhydrate ;

la réaction du produit de formule (I) dans laquelle R représente un atome d'hydrogène avec le dérivé halogéné de formule IV est effectuée au sein d'un alcanol tel que l'éthanol, au reflux du mélange, en présence d'une base aminée telle que la triéthylamine ;

la réaction du produit de formule (I) avec le produit de formule (V) est effectuée au sein d'un alcanol tel que l'éthanol, au reflux du mélange.

Selon une variante du procédé décrit ci-dessus, la réaction du produit de formule (I) dans laquelle R représente un atome d'hydrogène avec le dérivé halogéné de formule IV est effectuée à chaud, au sein d'une base aminée telle que le diméthylformamide et en présence d'iodure de potassium et d'un carbonate alcalin tel que le carbonate de potassium.

Les produits de formule (I) présentent un caractère basique.

On peut avantageusement préparer les sels d'addition des produits de formule (I) en faisant réagir, en proportions sensiblement stoéchiométriques, un acide minéral ou organique avec lesdits produits de formule (I). Les sels peuvent être préparés sans isoler les bases correspondantes.

Les produits, objet de la présente invention, possèdent de très intéressantes propriétés pharmacologiques ; ils sont notamment remarquables pour leurs propriétés antagonistes à la fois de la dopamine et des récepteurs alpha 2 et leur affinité généralement faible pour les récepteurs alpha 1 et muscarinique. Les produits selon l'invention possèdent ainsi des propriétés antidopaminergiques mais aussi antidépressives et/ou antipsychotiques et entraînent un minimum d'effets secondaires sur le système nerveux autonome et d'effets secondaires extrapyramidaux.

Ces propriétés sont illustrées plus loin dans la partie expérimentale.

Ces propriétés justifient l'utilisation des nouvelles benzothiopyrano pyridinones, objet de la présente invention, ainsi que de leurs sels pharmaceutiquement acceptables à titre de médicaments.

La présente demande a ainsi également pour objet l'application à titre de médicaments des nouvelles benzothiopyrano pyridinones telles que définies par la formule (I) ainsi que de leurs sels pharmaceutiquement acceptables.

Parmi les médicaments objet de l'invention, on retient de préférence les médicaments, caractérisés en ce qu'ils sont constitués par les dérivés répondant à la formule (I) dans laquelle A représente un atome d'hydrogène et B représente un atome d'hydrogène, un radical méthyle, méthoxy, un radical nitro ou acétamido ou un atome de chlore ou de fluor ou B forme avec A et les atomes de carbone auxquels ils sont liés un cycle benzénique, C représente un atome d'hydrogène ou un radical méthoxy et D représente

un atome d'hydrogène ou forme avec C et les atomes de carbone auxquels ils sont liés un cycle benzénique, R a la signification déjà indiquée, et leurs sels pharmaceutiquement acceptables.

Parmi ceux-ci, on retient notamment les médicaments, caractérisés en ce qu'ils sont constitués par les dérivés répondant à la formule (I) dans laquelle R représente un atome d'hydrogène ou un radical R' dans lequel R' représente un radical alcoyle linéaire ou ramifié, renfermant de 1 à 9 atomes de carbone, un radical benzyle, un radical cyclohexylméthyle ou un groupement p-fluorophényl-4-oxo butyl ou éthoxy carbonyléthyle, et leurs sels pharmaceutiquement acceptables.

Parmi les médicaments de l'invention on retient tout particulièrement ceux caractérisés en ce que A, B, C et D représentent un atome d'hydrogène, ainsi que leurs sels pharmaceutiquement acceptables et notamment les produits dont les noms suivent :

la 1,2,3,4-tétrahydro 2-n-propyl-10H-/1/-benzothiopyrano /3,2-c/ pyridin-10-one et ses sels ;
la 1,2,3,4-tétrahydro 2-n-butyl-10H-/1/-benzothiopyrano /3,2-c/ pyridin-10-one et ses sels ;
la 1,2,3,4-tétrahydro 2-isopentyl-10H-/1/-benzothiopyrano /3,2-c/pyridin-10-one et ses sels ;
la 1,2,3,4-tétrahydro 2-n-pentyl-10H-/1/-benzothiopyrano /3,2-c/ pyridin 10-one et ses sels ;
la 1,2,3,4-tétrahydro 2-(4-p-fluorophényl-4-oxobutyl)-10H-/1/-benzothiopyrano /3,2-c/ pyridin-10-one et ses sels ;
la 1,2,3,4-tétrahydro 2-cyclohexylméthyl 8-fluoro 10H-/1/-benzothiopyrano /3,2-c/ pyridin-10-one et ses sels ;
la 1,2,3,4-tétrahydro 2-cyclopropylméthyl 10H-/1/-benzothiopyrano /3,2-c/ pyridin-10-one et ses sels ;
la 1,2,3,4-tétrahydro 2-cyclobutylméthyl 10H-/1/-benzothiopyrano /3,2-c/ pyridin-10-one et ses sels ;
la 1,2,3,4-tétrahydro 2-cyclopentylméthyl 10H-/1/-benzothiopyrano /3,2-c/ pyridin-10-one et ses sels et, tout particulièrement
la 1,2,3,4-tétrahydro 2-cyclohexylméthyl 10H-/1/-benzothiopyrano /3,2-c/ pyridin-10-one et ses sels.

Ces médicaments trouvent par exemple leur emploi dans le traitement du déséquilibre neurovégétatif, des dépressions, des troubles caractériels, des troubles du comportement et des manifestations d'anxiété des états névrotiques.

La dose usuelle variable selon le produit utilisé, le sujet traité et l'affection en cause peut être, par exemple, de 0,25 mg à 1 000 mg par jour, et de préférence de 0,25 à 400 mg par jour par voie orale chez l'homme.

L'invention a ainsi pour objet les compositions pharmaceutiques qui renferment au moins un dérivé précité ou l'un de ses sels pharmaceutiquement acceptables, à titre de principe actif.

A titre de médicaments, les dérivés répondant à la formule I et leurs sels pharmaceutiquement acceptables peuvent être incorporés dans des compositions pharmaceutiques destinées à la voie digestive ou parentérale.

Ces compositions pharmaceutiques peuvent être par exemple solides ou liquides et se présenter sous les formes pharmaceutiques couramment utilisées en médecine humaine, comme par exemple, les comprimés, simples ou dragéifiés, les gélules, les granulés, les suppositoires, les sirops, les aérosols, les préparations injectables ; elles sont préparées selon les méthodes usuelles. Le ou les principes actifs peuvent y être incorporés à des excipients habituellement employés dans ces compositions pharmaceutiques, tels que le talc, la gomme arabique, la lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Les produits de formule (III), utilisés au départ du procédé de l'invention sont connus dans le cas où R représente un atome d'hydrogène. Dans le cas où R est différent d'hydrogène, les produits de formule (III) peuvent être préparés par exemple par cyclisation d'un produit de formule (VI) :

$$R—N\ [(CH_2)_2—COOX]_2 \qquad (VI)$$

dans laquelle R et X sont définis comme ci-dessus, R étant différent d'hydrogène. Cette cyclisation peut par exemple être effectuée en présence d'hydrure de sodium, dans un solvant tel que le toluène, renfermant une trace d'alcool, par exemple d'éthanol, l'élimination du solvant et de l'alcool étant assurée par chauffage et distillation.

Les produits de formule (VI) peuvent être préparés, par exemple, par action d'une amine appropriée de formule $R—NH_2$, dans laquelle R est défini comme ci-dessus, à l'exception d'hydrogène, avec deux équivalents d'un acrylate de formule (VII) :

$$CH_2 = CH—COOX \qquad (VII)$$

en opérant à chaud, en présence d'un solvant tel que l'éthanol.

Les exemples suivants illustrent l'invention sans toutefois la limiter.

Exemple 1

Chlorhydrate de 1,2,3,4-tetrahydro-10H-(1)-benzothiopyrano /3,2-c/pyridin-10-one.

On chauffe à 100 °C sous agitation, 35 g d'acide polyphosphorique et y ajoute lentement 2,33 g de chlorhydrate de 4-oxo 3-pipéridine carboxylate d'éthyle et 1,1 $cm^3$ de thiophénol.

On agite le mélange à 100 °C pendant quatre heures puis le verse dans 500 $cm^3$ d'eau, ajoute du carbonate de sodium jusqu'à l'obtention de pH = 8 et extrait au chloroforme. On lave les phases organiques à l'eau, les sèche, évapore le solvant dissout le résidu dans un peu de chloroforme et acidifie par addition d'une solution d'acide chlorhydrique dans l'éther.

On rajoute de l'éther, filtre le précipité obtenu, recristallise dans du méthanol aqueux et le sèche. (méthode A).

On obtient 1,365 g de produit attendu (rendement 54 %),

F = 270-272 °C (décomposition).

Exemples 2 à 10

En utilisant une méthode analogue à celle utilisée pour l'exemple 1, mais en partant du produit correspondant de formule II dans laquelle A, B, C, et D ont les significations indiquées dans le tableau ci-dessous, et des produits correspondants de formule III dans laquelle R a la signification indiquée dans le tableau I ci-dessous, on a préparé les produits des exemples 2 à 10 (voir Tableau I ci-après).

Les points de fusion et les microanalyses sont donnés dans le tableau II.

Exemple 2 : Chlorhydrate de 1,2,3,4-tétrahydro 8-méthyl-10H-(1)-, benzothiopyrano /3,2-c/pyridin-10-one.

Exemple 3 : Chlorhydrate de 1,2,3,4-tetrahydro 7-méthoxy-10H-(1)-benzothiopyrano /3,2-c/pyridin-10-one.

Exemple 4 : Chlorhydrate de 1,2,3,4-tetrahydro 8-méthoxy-10H-(1)-benzothiopyrano /3,2-c/pyridin-10-one.

Exemple 5 : Chlorhydrate de 1,2,3,4-tetrahydro 12H-naphtho/1',2',5,6/thiopyrano/3,2-c/pyridin-12-one.

Exemple 6 : Chlorhydrate de 2-benzyl-1,2,3,4-tetrahydro-10H-(1)-benzothiopyrano /3,2-c/pyridin-10-one.

Exemple 7 : Chlorhydrate de 8-chloro 1,2,3,4-tetrahydro-10H-(1)-benzothiopyrano/3,2-c/pyridin-10-one.

Exemple 8 : Chlorhydrate de 1,2,3,4-tétrahydro 12H-naphtho /1',2',6,5/thiopyrano/3,2-c/pyridin-12-one.

Exemple 9 : Chlorhydrate de 1,2,3,4-tétrahydro 8-nitro-10H-(1)-benzothiopyrano/3,2-c/pyridin-10-one.

Exemple 10 : Chlorhydrate de 8-acétamido 1,2,3,4-tétrahydro-10H (1)-benzothiopyrano/3,2-c/pyridin-10-one.

Exemple 11

Chlorhydrate de 1,2,3,4-tétrahydro 2-n-propyl-10H-(1)-benzothiopyrano /3,2-c/pyridin-10-one.

On ajoute goutte à goutte en vingt minutes une solution de 1,44 $cm^3$ d'iodure de n-propyl dans 10 $cm^3$ d'éthanol, à une solution portée au reflux de 2,5 g de chlorhydrate de 1,2,3,4-tétrahydro 10H-(1)-benzothiopyrano /3,2-c/ pyridin-10-one et 5 $cm^3$ de triéthylamine dans 100 $cm^3$ d'éthanol.

On maintient au reflux pendant quatre heures, au terme desquelles une chromatographie sur couche mince (silice, $CH_2CL_2$ + 10 % MeOH) indique que le produit de départ a disparu.

On évapore le solvant, ajoute au résidu du chloroforme et de l'acide chlorhydrique dilué.

La phase aqueuse est baséifiée a pH 8 par addition de carbonate de sodium, extraite au chloroforme.

Les phases organiques sont lavées à l'eau, séchées et évaporées à sec. Le résidu est purifié par chromatographie sur silice en éluant au chlorure de méthylène à 1 % de méthanol. Le produit solide obtenu est dissous dans le chloroforme, puis acidifié par addition d'une solution d'acide chlorhydrique dans l'éther. On rajoute de l'éther, filtre le précipité ainsi obtenu, le recristallise dans l'éthanol et le sèche (méthode $B_1$).

On obtient 1,75 g de produit attendu (rendement 60 %).

F = 218-221 °C.

Exemples 12 à 18

En utilisant une méthode analogue à celle utilisée à l'exemple 11, mais en utilisant au départ un composé correspondant de formule $I_A$ dans laquelle A, B, C et D ont les significations indiquées dans le tableau I ci-dessous, et R représente un atome d'hydrogène, et du composé correspondant de formule IV dans laquelle R' a la signification indiquée pour R dans le tableau I ci-dessous, on a préparé les composés des exemples 12 à 18. (voir tableau I ci-dessous).

Les points de fusion et les microanalyses sont indiqués dans le tableau II.

Exemple 12 : -Chlorhydrate de 1,2,3,4-tétrahydro-2-méthyl-10H-(1)-benzothiopyrano/3,2-c/pyridin-10-one.

Exemple 13 : Chlorhydrate de 2-éthyl-1,2,3,4-tétrahydro-10H-(1)-benzothiopyrano/3,2-c/pyridin-10-one.

Exemple 14 : Chlorhydrate de 2-n-butyl-1,2,3,4-tétrahydro-10H-(1) benzothiopyrano/3,2-c/pyridin-10-one.

Exemple 15 : Chlorhydrate de 1,2,3,4-tétrahydro -2-isopentyl-10H-(1)-benzothiopyrano/3,2-c/pyridin-10-one.

Exemple 16 : Chlorhydrate de 2-(4-p-fluorophényl-4-oxobutyl)-1,2,3,4-tétrahydro-10H-(1)-benzothiopyrano/3,2-c/pyridin-10-one.

Exemple 17 : Chlorhydrate de 1,2,3,4-tétrahydro 2-n-pentyl-10H-(1)-benzothiopyrano/3,2-c/pyridin-10-one.

Exemple 18 : Chlorhydrate de 2-n-hexyl-1,2,3,4-tétrahydro-10H-(1)-benzothiopyrano/3,2-c/pyridin-10-one.

### Exemple 19

Chlorhydrate de 1,2,3,4-tétrahydro-2-isobutyl-10H-(1)-benzothiopyrano/3,2-c/pyridin-10-one.

On ajoute en trente minutes à 100 °C, une solution de 1,58 $cm^3$ de bromure d'isobutyle dans 10 $cm^3$ de diméthylformamide à un mélange de 2,46 g de chlorhydrate de 1,2,3,4-tétrahydro-10H(1)-benzothiopyrano/3,2-c/pyridin-10-one, 2,81 g de carbonate anhydre de potassium et 0,1 g d'iodure de potassium dans 100 $cm^3$ de diméthyl formamide. On agite le mélange à 100 °C pendant six heures puis le verse dans 500 $cm^3$ d'eau et extrait à l'acétate d'éthyle. La phase organique est concentrée puis acidifiée par un mélange d'acide chlorhydrique et d'éther. On rajoute de l'éther, filtre le précipité obtenu et le recristallise dans l'éthanol puis le sèche. (méthode $B_2$).

On obtient 1,32 g de produit attendu (rendement 44 %)

F = 202-203 °C (décomposition).

### Exemple 20

Chlorhydrate de 2-cyclohexylméthyl-1,2,3,4-tétrahydro-10H-(1)-benzothiopyrano/3,2-c/pyridin-10-one.

En utilisant une méthode analogue à celle utilisée à l'exemple 19 mais en partant de chlorhydrate de 1,2,3,4-tétrahydro-10H-(1)-benzopyrano/3,2-c/pyridin-10-one et de bromométhyl cyclohexane, on obtient le produit attendu (voir tableau I ci-dessous).

Le point de fusion et la microanalyse sont indiqués dans le tableau II.

### Exemple 21

Chlorhydrate de 2-(2-éthoxycarbonyléthyl)-1,2,3,4-tétrahydro-10H-(1)-benzothiopyrano/3,2-c/pyridin-10-one.

On porte au reflux pendant vingt heures un mélange de 2 g de 1,2,3,4-tétrahydro 10H-(1)-benzothiopyrano/3,2-c/pyridin-10-one (obtenu à partir du chlorhydrate par traitement basique) et 0,95 $cm^3$ d'acrylate d'éthyle dans 60 $cm^3$ d'éthanol. On évapore le mélange obtenu, dissout l'huile résiduelle dans le chlorure de méthylène et acidifie par un mélange d'acide chlorhydrique et d'éther. On rajoute de l'éther, filtre le précipité obtenu et le recristallise dans l'éthanol puis le sèche. (méthode C).

On obtient 1,54 g de produit attendu (rendement 55 %)

F = 190-192,5 °C (décomposition)

### Exemples 22 et 23

En utilisant une méthode analogue à celle utilisée à l'exemple 21, mais en partant de 1,2,3,4-tétrahydro 10H-(1)-benzothiopyrano/3,2-c/pyridin-10-one et du produit correspondant de formule V, on a préparé les composés des exemples 22 et 23 (voir tableau I ci-dessous) ;

Le point de fusion et les microanalyses sont indiqués dans le tableau II.

Exemple 22 : Chlorhydrate de 2-(2-cyanoéthyl)-1,2,3,4-tétrahydro-10H-(1)-benzothiopyrano/3,2-c/pyridin-10-one.

Exemple 23 : Chlorhydrate de 2-(2-carbamoyléthyl)-1,2,3,4-tétrahydro-10H-(1)-benzothiopyrano/3,2-c/pyridin-10-one.

### Exemple 24

Chlorhydrate de 2-cyclohexylméthyl-8-fluoro-1,2,3,4-tétrahydro-10H-(1)-benzothiopyrano/3,2-c/pyridin-10-one.

Stade 1 : 3,3'-cyclohexylméthylamino bispropionate de diéthyle.

On dissout 21,75 g de cyclohexylméthylamine dans 100 $cm^3$ d'éthanol puis ajoute lentement 57,7 g d'acrylate d'éthyle et porte le mélange au reflux pendant vingt heures.

Le solvant et l'excès d'acrylate d'éthyle sont évaporés sous vide puis l'huile résiduelle est distillée sous pression réduite.

On obtient 50,4 g (rendement 84 %) de produit attendu.

Eb. 154-156 °C sous 0,5 mm de mercure.

Stade 2 : Chlorhydrate de 1-cyclohexylméthyl-4-oxopipéridin-3-carboxylate d'éthyle.

On dissout 39 g de produit obtenu au stade 1 dans 250 $cm^3$ de toluène, puis ajoute sous azote sous agitation, 5 g d'hydrure de sodium à 80 % dans l'huile minérale, puis quelques gouttes d'éthanol.

Le mélange obtenu est chauffé lentement pour distiller le mélange éthanol-toluène, jusqu'à ce que la température du distillat atteigne 110 °C. On refroidit à température ambiante puis ajoute lentement sous agitation 15 $cm^3$ d'acide chlorhydrique concentré.

On ajoute un minimum d'eau pour dissoudre le précipité de chlorure de sodium formé et sépare la phase organique.

La phase aqueuse est extraite à l'éther puis les phases organiques réunies sont lavées à l'eau et séchées.

On y ajoute 50 $cm^3$ d'une solution à 10 % d'acide chlorhydrique dans l'éthanol afin de précipiter le produit sous forme de chlorhydrate. Ce dernier est filtré, lavé à l'éther et séché.

On obtient 25 g (rendement 66 %) de produit attendu.

F = 153 °C.

Stade 3 : Chlorhydrate de 2-cyclohexylméthyl 8-fluoro 1,2,3,4-tétrahydro-10H-(1)-benzothiopyrano/3,2-c/pyridin-10-one.

On chauffe 80 g d'acide polyphosphorique à 100 °C-120 °C puis ajoute sous agitation en vingt minutes, 6,68 g de produit obtenu au stade 2 et 2,51 g de parafluorothiophénol, chacune de ces deux additions se faisant en deux fois.

La température est maintenue à 110 °C pendant deux heures. La solution jaune résultante est versée dans l'eau puis on y ajoute jusqu'à l'obtention de pH 8, du carbonate de sodium. Le précipité obtenu est filtré, lavé à l'eau puis dissous dans le chlorure de méthylène. On lave la solution à l'eau, la sèche et évapore le solvant. On dissout le résidu dans un mélange d'acétate d'éthyle et d'éthanol puis on y ajoute 10 $cm^3$ d'une solution à 10 % d'acide chlorhydrique dans l'éthanol afin de précipiter le sel. Ce dernier est filtré, lavé à l'éthanol, et séché. On obtient 2,73 g de produit attendu (rendement 39 %)

F = 243 °C (décomposition).

Analyse : $C_{19}H_{23}NOSClF$

Calculé : C % 62,03 H 6,30 N 3,81 Cl 9,64 S 8,71 F 5,16 %

Trouvé : C % 61,75 H 6,37 N 3,74 Cl 9,83 S 8,72 F 5,07 %

Exemples 25 à 37

Les composés de formule I dans laquelle A = B = C = D = H et dans laquelle le groupe R est identifié comme décrit dans le tableau III ci-après ont été préparés à partir des produits de départ appropriés.

Les procédés utilisés sont analogues à ceux des exemples 11 à 19 (méthode B).

Le produit de l'exemple 37 a été préparé par une méthode analogue à celle utilisée à l'exemple 24.

Les constantes obtenues pour ces produits sont indiquées dans le tableau III ci-après.

(Voir Tableaux 1, 2, 3 et 4 p. 16 et suiv.)

Tableau I (partie 1)

| Exemple | A | B | C | D | R | Méthode de synthèse. |
|---|---|---|---|---|---|---|
| 1 | H | H | H | H | H,HCl | A |
| 2 | H | Me | H | H | H,HCl | A |
| 3 | H | H | OMe | H | H,HCl | A |
| 4 | H | OMe | H | H | H,HCl | A |
| 5 | 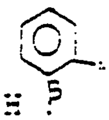 | | H | H | H,HCl | A |
| 6 | H | H | H | H | $CH_2$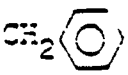.HCl | A |
| 7 | H | Cl | H | H | H,HCl | A |
| 8 | H | H | 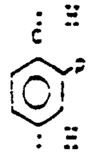 | | H,HCl | A |
| 9 | H | $NO_2$ | H | H | H,HCl | A |
| 10 | H | NHAC | H | H | H,HCl | A |
| 11 | H | H | H | H | $CH_2CH_2CH_3$,HCl | $B_1$ |
| 12 | H | H | H | H | $CH_3$,HCl | $B_1$ |
| 13 | H | H | H | H | $CH_2CH_3$,HCl | $B_1$ |
| 14 | H | H | H | H | $CH_2CH_2CH_2CH_3$,HCl | $B_1$ |
| 15 | H | H | H | H | $CH_2CH_2CH(CH_3)_2$,HCl | $B_1$ |
| 16 | H | H | H | H | $CH_2CH_2CH_2$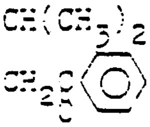F,HCl | $B_1$ |
| 17 | H | H | H | H | $CH_2CH_2CH_2CH_2CH_3$,HCl | $B_1$ |
| 18 | H | H | H | H | $CH_2CH_2CH_2CH_2CH_2CH_3$,HCl | $B_1$ |
| 19 | H | H | H | H | $CH_2CH(CH_3)_2$,HCl | $B_2$ |
| 20 | H | H | H | H | $CH_2$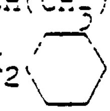,HCl | $B_2$ |
| 21 | H | H | H | H | $CH_2CH_2CO_2Et$;HCl | C |
| 22 | H | H | H | H | $CH_2CH_2CN$,HCl | C |
| 23 | H | H | H | H | $CH_2CH_2CONH_2$,HCl | C |

Tableau I (partie 2)

| Exemple | Rendement % | Solvant de recristallisation | IR ($cm^{-1}$) KBr |
|---|---|---|---|
| 1 | 55 | MeOH/$H_2O$ | 3420br,2800br,1610,1590,1440, 1395,1180,1080,1020,740 |
| 2 | 65 | MeOH | 3440br,2750br,1620,1600,1415, 1335,1105,825,765 |
| 3 | 61 | MeOH/$H_2O$ | 3430br,2740br,1615,1595,1420, 1240, 1035,850,760 |
| 4 | 58 | MeOH | 3520br,2740br,1600br,1485, 1425,1220,1125,825,765 |
| 5 | 59 | MeOH | 3500br,2760br,1595br,1423, 1395,1120,820,795,745 |
| 6 | 53 | EtOH/$H_2O$ | 3410br,2390br,1620,1595,1440, 1380,745 |
| 7 | 43 | MeOH/$H_2O$ | 3450br,2750br,1610,1590,1415, 1330,1095,825,765 |
| 8 | 60 | MeOH/$H_2O$ | 3400br, 2800br,1605,1580,1400 1190,820,760 |
| 9 | 10 | EtOH/$H_2O$ | 3500br,2960,1625,1610,1530, 1345,738 |
| 10 | | MeOH/$H_2O$ | |
| 11 | 60 | EtOH | 3450br,2470br,1625,1595,1440, 1385,1330,985,795,740 |
| 12 | 37 | EtOH | 3500br,2500br,1620,1580br, 1480,1435,1395,1330,1195,1080 995,820,790,745,730 |
| 13 | 52 | EtOH | 3400br,2500br,1615,1595,1445, 1325,740,735 |
| 14 | 43 | EtOH | 3480br,2500br,1620,1595,1440, 1385,1325,740 |
| 15 | 45 | EtOH | 3450br,2500br,1620br,1390, 1320,740,1440 |
| 16 | 25 | | 3450br,2440br,1690,1600br, 1215, 1155,975,740 |
| 17 | 51 | EtOH/EtOAC | 3500br,2500br,1630,1600,1445, 1390,1330,950,745 |
| 18 | 57 | EtOH/EtOAC | 3500br,2950,2400br,1630,1595, 1445br,1390,1330,795,745 |
| 19 | 44 | EtOH/$H_2O$ | 3500br,2500br,1620,1590,1440, 1420,1385,1325,980,735 |
| 20 | 35 | EtOH | 3500br,2450br,1630,1595,1445, 1385,1325,980,745 |
| 21 | 55 | EtOH | 3450br,2500br,1745,1620,1595, 1445,1390,1325,1200,1080,1030 745 |
| 22 | 63 | EtOH/$H_2O$ | 3500br,2460br,2230,1620,1595 1475,1430br,1390,1330,970,745 |
| 23 | 60 | MeOH/$H_2O$ | 3350br,2550br,1675,1620,1590 1445,1425,1400,1325,745 |

Tableau II

| Ex. | F°C | Formule | Poids moléculaire incluant $H_2O$ | Moles $H_2O$ | Calculé C | Calculé H | Calculé N | Calculé S | Calculé Cl | Trouvé C | Trouvé H | Trouvé N | Trouvé S | Trouvé Cl |
|---|---|---|---|---|---|---|---|---|---|---|---|---|---|---|
| 1 | 270-272 | $C_{12}H_{12}NOSCl$ | 253.75 | - | 56,80 | 4,77 | 5,52 | 12,63 | 13,97 | 56,76 | 4,74 | 5,67 | 12,61 | 13,91 |
| 2 | 268-269.5 | $C_{13}H_{14}NOSCl$ | 267.77 | - | 58.31 | 5.27 | 5.23 | 11.97 | 13.24 | 58.00 | 5.26 | 5.28 | 12.01 | 13.21 |
| 3 | 269-271 | $C_{13}H_{14}NO_2SCl$ | 283.77 | - | 55.02 | 4.97 | 4.94 | 11.30 | 12.49 | 55.08 | 4.94 | 4.92 | 11.45 | 12.56 |
| 4 | 241-242 | $C_{13}H_{14}NO_2SCl$ | 283.77 | - | 55.02 | 4.97 | 4.94 | 11.30 | 12.49 | 54.87 | 5.02 | 4.92 | 11.16 | 12.47 |
| 5 | 226-229 | $C_{16}H_{14}NOSCl$ | 312.82 | 0.5 | 61.43 | 4.83 | 4.48 | 10.25 | 11.33 | 61.84 | 4.79 | 4.67 | 10.35 | 11.33 |
| 6 | 219-222 | $C_{19}H_{18}NOSCl$ | 343.87 | - | 66.37 | 5.28 | 4.07 | 9.32 | 10.31 | 66.31 | 5.31 | 3.95 | 9.29 | 10.29 |
| 7 | 281-285 | $C_{12}H_{11}NOSCl_2$ | 297.21 | 0.5 | 48.49 | 4.07 | 4.71 | 10.79 | 23.86 | 48.55 | 4.02 | 4.74 | 10.93 | 23.91 |
| 8 | 245-248 | $C_{16}H_{14}NOSCl$ | 321.83 | 1.0 | 59.71 | 5.01 | 4.35 | 9.96 | 11.02 | 60.04 | 5.04 | 4.48 | 10.00 | 11.25 |
| 9 | | $C_{12}H_{11}N_2O_3SCl$ | | | | | | | | | | | | |
| 10 | | $C_{14}H_{15}N_2O_2SCl$ | | | | | | | | | | | | |
| 11 | 218-221 | $C_{15}H_{18}NOSCl$ | 295.83 | - | 60.90 | 6.13 | 4.74 | 10.84 | 11.98 | 60.60 | 6.09 | 4.69 | 10.67 | 11.99 |
| 12 | 237-238 | $C_{13}-H_{14}NOSCl$ | 276.78 | 0.5 | 56.41 | 5.46 | 5.06 | 11.58 | 12.81 | 56.41 | 5.45 | 5.17 | 11.70 | 13.02 |
| 13 | 221-224 | $C_{14}H_{16}NOSCl$ | 281.81 | - | 59.67 | 5.72 | 4.97 | 11.38 | 12.58 | 59.57 | 5.70 | 5.01 | 11.53 | 12.70 |
| 14 | 209-211 | $C_{16}H_{20}NOSCl$ | 318.87 | 0.5 | 60.27 | 6.64 | 4.39 | 10.05 | 11.12 | 60.45 | 6.62 | 4.24 | 10.29 | 11.10 |
| 15 | 196-199 | $C_{17}H_{22}NOSCl$ | 328.39 | 0.25 | 62.18 | 6.91 | 4.27 | 9.76 | 10.80 | 62.13 | 6.85 | 4.19 | 9.73 | 10.83 |
| 16 | 236-239 | $C_{22}H_{21}NO_2SClF$ | | | | | | | | | | | | |
| 17 | 182-185 | $C_{17}H_{22}NOSCl$ | 328.39 | 0.25 | 62.18 | 6.91 | 4.27 | 9.76 | 10.80 | 62.17 | 6.90 | 4.21 | 9.70 | 10.92 |
| 18 | 175-177 | $C_{18}H_{24}NOSCl$ | 324.42 | 0.25 | 63.14 | 7.21 | 4.09 | 9.36 | 10.39 | 63.48 | 7.15 | 4.03 | 9.35 | 10.39 |
| 19 | 202-203 | $C_{16}H_{20}NOSCl$ | 309.86 | - | 62.02 | 6.51 | 4.52 | 10.35 | 11.44 | 61.71 | 6.45 | 4.52 | 10.56 | 11.50 |
| 20 | 171-173 | $C_{19}H_{24}NOSCl$ | 349.92 | - | 65.22 | 6.91 | 4.00 | 9.16 | 10.13 | 64.92 | 6.79 | 4.00 | 9.31 | 10.28 |
| 21 | 190-192.5 | $C_{17}H_{20}NO_3SCl$ | 367.39 | 0.75 | 55.58 | 5.90 | 3.81 | 8.73 | 9.65 | 55.54 | 5.87 | 3.79 | 9.18 | 9.85 |
| 22 | 247-252 | $C_{15}H_{15}N_2OSCl$ | 306.81 | - | 58.72 | 4.93 | 9.13 | 10.45 | 11.59 | 58.89 | 4.95 | 9.13 | 10.49 | 11.66 |
| 23 | 249-253 | $C_{15}H_{17}N_2O_2SCl$ | 324.83 | - | 55.47 | 5.28 | 8.62 | 9.87 | 10.91 | 55.23 | 5.29 | 8.48 | 9.89 | 10.96 |

Tableau III

| Ex No. | R | F°C | Calculé C | Calculé H | Calculé N | Calculé Cl | Calculé S | Trouvé C | Trouvé H | Trouvé N | Trouvé Cl | Trouvé S |
|---|---|---|---|---|---|---|---|---|---|---|---|---|
| 25 | 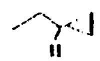 HCl.0.33$H_2O$ | 257°(dec) | 61.24 | 5.99 | 4.46 | 11.30 | 10.21 | 61.11 | 6.02 | 4.37 | 11.31 | 10.21 |
| 26 | 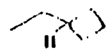 HCl | 244 (dec) | 63.44 | 6.26 | 4.35 | 11.01 | 9.96 | 63.19 | 6.24 | 4.27 | 10.97 | 9.85 |
| 27 | 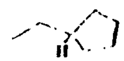 HCl | 240 (dec) | | | | | | | | | | |
| 28 | 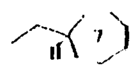 HCl | 241 (dec) | 65.20 | 7.25 | 3.80 | 9.62 | 8.70 | 65.32 | 7.22 | 3.78 | 9.81 | 8.88 |
| 29 | 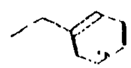 HCl | 256 (dec) | 65.59 | 6.38 | 4.03 | 10.19 | 9.22 | 64.96 | 6.35 | 4.01 | 10.35 | 9.17 |
| 30 | 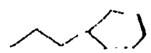 Free base | 115° | 73.35 | 7.69 | 4.28 | - | 9.79 | 73.49 | 7.69 | 4.21 | - | 9.81 |
| 31 | 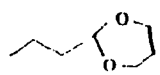 Free base | 99° | 65.23 | 6.39 | 4.23 | - | 9.67 | 65.20 | 6.37 | 4.24 | - | 9.80 |
| 32 | 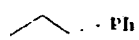 HCl | 238 (dec) | 65.47 | 5.77 | 3.82 | 9.66 | 8.74 | 65.85 | 5.72 | 3.84 | 10.01 | 8.93 |
| 33 | 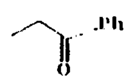 HCl | 255 (dec) | 64.60 | 4.88 | 3.77 | 9.53 | 8.62 | 64.37 | 4.96 | 3.71 | 9.51 | 8.57 |
| 34 | 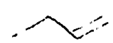 HCl.0.25$H_2O$ | 257 (dec) | 60.39 | 5.58 | 4.70 | 11.88 | 10.75 | 60.41 | 5.65 | 4.59 | 11.87 | 10.63 |
| 35 | 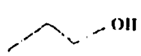 HCl.0.25$H_2O$ | 239° | 55.62 | 5.50 | 4.63 | 11.73 | 10.60 | 55.74 | 5.49 | 4.53 | 11.63 | 10.60 |
| 36 | 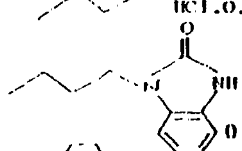 HCl 0.5$H_2O$ | 265° (dec) | 60.47 | 5.30 | 9.62 | 8.11 | 7.34 | 60.69 | 5.17 | 9.62 | 8.27 | 7.30 |
| 37 | 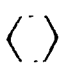 HCl | 243° (dec) | 64.35 | 5.60 | 4.17 | 10.55 | 9.54 | 64.13 | 6.64 | 4.09 | 10.63 | 9.34 |

Exemple 38

Chlorhydrate de 2-cyclohexylmethyl 8-méthoxy 1,2,3,4-tétrahydro 10H-/1/-benzothiopyrano /3,2-c/ pyridin-10-one.

En utilisant une méthode analogue à celle décrite à l'exemple 24, mais en utilisant le p-méthoxythiophénol au lieu du p-fluorothiophénol, on obtient le produit attendu. F = 231 °C (décomposition).
I.R. (KBr) 2 920 $cm^{-1}$, 2 840 $cm^{-1}$, 2 300-2 600 $cm^{-1}$, 1 690 $cm^{-1}$
RMN (base libre — $CDCl_3$)

| | | | |
|---|---|---|---|
| 2,1 | 1H | d (J = 2,5 Hz) | $H_9$ |
| 2,67 | 1H | d (J = 9 Hz) | $H_6$ |
| 2,9 | 1H | dd (J = 2,5 and 9 Hz) | $H_7$ |
| 6,14 | 3H | s | OMe |
| 6,48 | 2H | bs | $2xH_1$ |
| 7,37 | 4H | bs | $2x(H_3+H_4)$ |
| 7,65 | 2H | bd (J = 6 Hz) | $N-CH_2$ |
| 7,9-9,5 | 11H | m | cyclohexyl |

Analyse : $C_{20}H_{26}NClSO_2$
Calculé : C 63,22 H 6,90 N 3,69 Cl 9,33 S 8,44 %
Trouvé : C 63,03 H 6,91 N 3,64 Cl 9,43 S 8,51 %

Exemple 39

On a préparé les comprimés selon la formulation :

| | |
|---|---|
| Composé de l'exemple 20 | 25 mg |
| Excipient q.s. pour un comprimé terminé à | 150 mg |

(Détail de l'excipient : lactose, amidon, talc, stéarate de magnésium.)

Exemple 40

On a préparé les comprimés selon la formulation :

| | |
|---|---|
| Composé de l'exemple 16 | 25 mg |
| Excipient q.s. pour un comprimé terminé à | 150 mg |

(Détail de l'excipient : lactose, amidon, talc, stéarate de magnésium).

Activité pharmacologique

Test A

Liaison de [$^3$H] spiroperidol aux récepteurs « dopaminergiques » sur des membranes du striatum des rats.

La liaison de [$^3$H] spiroperidol sur les membranes du striatum du rat présente une composante du type dopaminergique (Leysen, J. E. et al. Nature. 272, 168-171 (1978)). Les activités obtenues au niveau des membranes du striatum donnent une bonne indication de l'effet antidopaminergique des composés neuroleptiques.

Matériel et méthode.

Ligand radioactif : le /phényl-4(n)-$^3$H spiroperidol (Amersham International, TRK 570, 21 Ci/mmol) est utilisé, conservé en solution dans l'éthanol (1 µCi/1 µl). Cette solution est diluée dans de l'éthanol (250 µCi(1 ml) et cette solution-stock est diluée dans un tampon (3,2/10 000). L'essai est réalisé avec 0,1 ml de cette solution (le volume final du milieu de l'incubation étant de 2 ml).
= 0,19 nM de [$^3$H] spiroperidol
= 0,08 µCi/par échantillon.
Tampon.
Le tampon utilisé est un tampon Tris HCl 50 mM pH 7,6 contenant les produits suivants:

| | |
|---|---|
| NaCl | 120 mM |
| KCl | 5 mM |
| $MgCl_2$ | 1 mM |
| $CaCl_2$ | 2 mM |
| Pargyline | 10 μM |
| Acide ascorbique | 0,1 % |

Préparation des membranes.

Le striatum est disséqué à partir du cerveau de 4 rats mâles (CFHB) selon la méthode de J. Glowinski et L. L. Iversen (Eur. J. Pharmacol. 49, 201-202 (1978). Le cortex frontal étant considéré comme le tissu cortical correspondant à la partie « C » (voir référence).

Les animaux sont sacrifiés par décapitation et les tissus après dissection sont homogénisés à 0 °C dans 20 volumes de sucrose 0,32 M avec un homogéniseur Teflon/verre. L'homogénat est centrifugé à 1 000 g pendant 10 minutes et le surnageant est recentrifugé à 30 000 g pendant 30 minutes. Le surnageant est éliminé et le culot est remis en suspension par homogénisation dans 30 volumes de tampon à 0 °C.

Liaison non spécifique.

La liaison non spécifique est évaluée en ajoutant du butaclamol froid (1 μm) aux échantillons blancs. On utilise le butaclamol en solution aqueuse à 20 μm.

Les composés à étudier.

Les solutions sont préparées dans de l'eau distillée à une concentration 20 fois supérieure à celle de la concentration finale par échantillon.

Protocole.

1,3 ml de tampon
0,5 ml de suspension membranaire
0,1 ml d'eau distillée ou de solution de composé
0,1 ml de [$^3$H] spiroperidol.

Les tubes sont conservés dans la glace. Après l'addition de [$^3$H] spiroperidol, les échantillons sont incubés à 37 °C pendant 10 minutes. Les membranes sont séparées par filtration sur des filtres Whatman GF/C sous vide, puis sont rincées par 2 × 10 ml de tampon à 0 °C. Les filtres sont séchés à 80 °C et la radioactivité est comptée dans 5 ml d'econofluor (NEN). Tous les échantillons sont réalisés en triple.

Les résultats du déplacement du [$^3$H] spiroperidol fixé sur les récepteurs des membranes striatales (test A) sont rassemblés dans le tableau ci-après. Les résultats sont exprimés en $CI_{50}$(nM). Ils sont obtenus graphiquement à partir du logarithme de la dose utilisée, par rapport au pourcentage d'inhibition de l'agent traceur spécifique.

Test B

Liaison de [$^3$H] prazosine aux récepteurs alpha 1 du cortex frontal des rats.

La liaison de [$^3$H] prazosine sur les membranes du cortex frontal du rat présente une composante principalement sérotoninergique. La capacité de certains composés à déplacer ce marqueur radioactif fixé sur des récepteurs du cortex frontal donne une bonne corrélation avec leurs effets sur d'autres modèles d'activité résotoninergique, aussi bien pour des agonistes que pour des antagonistes.

Matériel et méthode.

Ligand radioactif : La [$^3$H] prazosine (Amersham International TRK 647,28 Ci/mmol) est utilisée conservée en solution dans l'éthanol (1 μCi/1 μl). Cette solution-stock est diluée dans un tampon (1/18 000). L'essai est réalisé avec 0,1 ml de cette solution (le volume final du milieu de l'incubation étant de 2,1 ml).

= 0,1 nM de [$^3$H] prazosine
= 0,0056 μCi/par échantillon

Préparation des membranes.

Le cortex frontal du cerveau de 4 rats mâles (CFHB) est disséqué et homogénisé à 0 °C dans 20 volumes de sucrose 0,32 M avec un homogéniseur Teflon/verre. L'homogénat est centrifugé à 1 000 g pendant 10 minutes et le surnageant est recentrifugé à 30 000 g pendant 30 minutes. Le surnageant est éliminé et le culot est remis en suspension par homogénisation dans 50 volumes de tampon Tris HCl 50 mM pH 7,5.

Liaison non spécifique.

La liaison non spécifique est évaluée en ajoutant 100 µM de nor adrénaline aux échantillons blancs. On utilise une solution de 2 mM de nor adrénaline fraîchement préparée dans une solution à 0,1 % d'acide ascorbique.

Les composés à étudier.

Les solutions sont préparées dans de l'eau distillée à une concentration 20 fois supérieure à celle de la concentration finale par échantillon.

Protocole.

1,3 ml de tampon
0,5 ml de suspension membranaire
0,1 ml d'eau distillée ou de solution de composé
0,1 ml de solution de [$^3$H] prazosine.

Les tubes sont conservés dans la glace. Après l'addition de [$^3$H] prazosine, les échantillons sont incubés à 25 °C pendant 30 minutes. Les membranes sont séparées par filtration sur des filtres Whatman GF/C sous vide, puis sont rincés par 2 × 10 ml de tampon à 0 °C. Les filtres sont séchés à 80 °C et la radioactivité est comptée dans 5 ml d'econofluor (NEN). Tous les échantillons sont réalisés en triple.

Les résultats du déplacement de la [$^3$H] prazosine fixée sur les récepteurs du cortex frontal sont rassemblés dans le tableau ci-après. Les résultats sont exprimés en $CI_{50}$ (nM). Ils sont obtenus graphiquement à partir du logarithme de la dose utilisée par rapport au pourcentage d'inhibition de l'agent traceur spécifique.

Test C

Liaison aux récepteurs alpha 2 sur les membranes du cortex du rat à l'aide de [$^3$H] dihydroergocryptine.

Dans les membranes du cortex, la [$^3$H] dihydroergocryptine se lie aux récepteurs alpha 1 et alpha 2 dans les conditions du test ci-après, 80 % environ de la liaison étant faite avec les récepteurs alpha 2.

Matériel et méthode.

Ligand radioactif : La /9,10(n)-$^3$H/9,10-dihydroergocryptine (Amersham International TRK 555,17,5 Ci/mmol) est utilisée en solution à 1 µCi/µl. Cette solution est diluée à 1/6250 dans un tampon. 0,1 ml de la solution obtenue est ajouté à 2 ml de la solution à tester.

= 0,45 nM de [$^3$H] dihydroergocryptine
= 0,016 µCi/par échantillon

Préparation des membranes.

Voir test B ci-dessus.

Liaison non spécifique.

Voir test B ci-dessus.

Composés à tester.

Voir test B ci-dessus.

Protocole.

Il est le même que celui décrit au test B ci-dessus, sauf en ce qui concerne l'incubation qui est effectuées à 25 °C pendant 45 minutes, à l'abri de la lumière.

Les résultats du test C sont rassemblés dans le tableau ci-après.

Test D

Liaison aux récepteurs muscariniques sur des membranes du cortex du rat à l'aide du [$^3$H] quinuclidinyl benzylate.

Des sites de liaison à haute affinité pour le [$^3$H] quinuclidinyl benzylate sont présents dans des homogénats du cerveau de rat. Les caractéristiques de ces sites de liaison ressemblent à ceux des récepteurs muscariniques cholinergiques. Les agonistes et antagonistes muscariniques déplacent la liaison spécifique du [$^3$H] quinuclidinyl benzilate avec une intensité qui est en rapport avec la puissance de leur effet pharmacologique. Les effets anticholinergiques des antidépresseurs et neuroleptiques peuvent avoir des conséquences pour leur utilisation chez l'homme car ils entraînent une mauvaise tolérance de ces composés (Snyder et al. Arch. Gen. Psychiat. 34 (2) 326-329 (1977)).

Ligand radioactif : le DL (3-[$^3$H]) quinuclidinyl benzilate (Amersham International TRK 506, 12 Ci/mmol) est utilisé en solution à 1 µCi/µl. Cette solution est diluée dans du tampon (1/13333). L'essai est réalisé avec 0,1 ml de cette solution (le volume final du milieu de l'incubation étant de 2,1 ml).

= 0,31 nM de [$^3$H] quinuclidinyl benzilate
= 0,0075 µCi/par échantillon.

Préparation des membranes.

Elle est effectuée comme au test B.

Liaison non spécifique.

La liaison non spécifique est évaluée en ajoutant 100 µM de sesquifumarate d'oxotrémorine froide aux échantillons blancs. On utilise une solution 2 mM de sesquifumarate d'oxotrémorine dans de l'eau distillée.

Composés à tester.

Voir test B ci-dessus.

Protocole.

Il est identique à celui du test B ci-dessus, sauf en ce qui concerne l'incubation qui est effectuée pendant 60 minutes à 25 °C.

Les résultats du test D sont rassemblés dans le tableau ci-après.

| | $CI_{50}$ µM | | | |
|---|---|---|---|---|
| Exemple | Test récepteur A DA | B $\alpha_1$ | C $\alpha_2$ | D MUSC |
| 1 | 6.5 | 6.6 | 0.6 | 32.0 |
| 2 | > 10 | > 10 | 3.3 | 45.7 |
| 3 | > 10 | > 10 | 4.3 | 26.9 |
| 4 | > 10 | 7.8 | 2.9 | 57.5 |
| 5 | 5.2 | 1.7 | 1.6 | 19.5 |
| 6 | 0.68 | 4.0 | 0.5 | > 100 |
| 7 | 5.1 | > 10 | 2.2 | 85.1 |
| 8 | 0.68 | 0.74 | 1.0 | 8.1 |
| 11 | 0.49 | 1.3 | 0.15 | > 100 |
| 12 | 2.8 | 3.1 | 0.93 | 32.4 |
| 13 | 1.0 | 1.5 | 0.29 | 44.7 |
| 14 | 0.08 | 0.89 | 0.11 | 75.8 |
| 15 | 0.06 | 0.68 | 0.17 | 87.1 |
| 16 | 0.02 | 0.02 | 0.02 | > 100 |
| 17 | 0.07 | 0.31 | 0.07 | 39.8 |
| 18 | 0.13 | 0.40 | 0.08 | 15.9 |

Tableau (Suite)

| CI$_{50}$ μM | | | | |
|---|---|---|---|---|
| Exemple | Test récepteur A DA | B $\alpha_1$ | C $\alpha_2$ | D MUSC |
| 19 | 0.10 | 4.6 | 0.15 | > 100 |
| 20 | 0.01 | 2.7 | 0.23 | > 100 |
| 21 | 0.78 | 7.1 | 1.0 | 100 |
| 22 | >10 | > 10 | >10 | > 100 |
| 23 | 4.2 | 5.9 | 3.0 | > 100 |
| 24 | 0.04 | >10 | >10 | > 100 |
| 25 | 0.22 | 1.23 | 0.06 | 43.0 |
| 26 | 0.06 | 0.69 | 0.05 | 50.0 |
| 27 | 0.04 | 0.68 | 0.05 | 57.5 |
| 28 | 0.15 | 2.82 | 0.32 | > 100 |
| 29 | 0.17 | 2.63 | 0.15 | > 100 |
| 30 | 0.18 | 1.15 | 0.19 | > 100 |
| 31 | 0.56 | 3.40 | 0.65 | 74.1 |
| 32 | 0.23 | 0.17 | 0.21 | 46.8 |
| 33 | > 10 | > 10 | >10 | > 100 |
| 34 | 2.81 | 3.98 | 0.39 | 37.2 |
| 35 | 10 | 10.0 | 3.30 | 71.0 |
| 36 | 0.03 | 0.19 | 0.15 | 44.7 |
| 37 | 0.16 | 1.59 | 0.60 | 11.7 |
| 38 | 0.76 | >10 | 2.19 | 91.2 |

**Revendications** (pour les Etats contractants : BE, CH, DE, FR, IT, LI, LU, NL, SE)

1. Benzothiopyranopyridinones et leurs sels, caractérisées en ce qu'elles répondent à la formule :

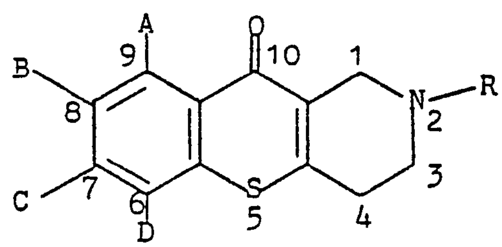

(I)

dans laquelle A, B, C et D, identiques ou différents, représentent un atome d'hydrogène, un radical alcoyle linéaire ou ramifié renfermant de 1 à 6 atomes de carbone, un radical alcoxy linéaire ou ramifié renfermant de 1 à 6 atomes de carbone, un radical nitro, un radical amino, un radical acylamino renfermant de 2 à 7 atomes de carbone ou un atome d'halogène, ou l'un des groupes A et B ou C et D représente avec les atomes de carbone auxquels ils sont liés, un cycle benzénique et l'autre est défini comme ci-dessus,

R représente un atome d'hydrogène ou un radical R' dans lequel R' représente un radical alcoyle, linéaire ou ramifié, renfermant de 1 à 9 atomes de carbone ou alcényle, linéaire ou ramifié, renfermant de 2 à 9 atomes de carbone, éventuellement substitué par un radical hydroxy, un radical cycloalcoyle renfermant de 3 à 9 atomes de carbone, un radical phényle, un radical aralcoyle renfermant de 7 à 9 atomes de carbone ou un groupement $-(CH_2)_n-Z$ dans lequel n est un nombre entier qui varie de 1 à 4 et Z représente un radical cycloalcoyle ou cycloalcényle renfermant de 3 à 9 atomes de carbone, un radical dioxanyl, un radical 2-oxo benzimidazolyl, un radical benzoyl éventuellement substitué par un atome d'halogène, un radical alcoxycarbonyl renfermant de 2 à 7 atomes de carbone, un radical cyano ou un radical carbamoyl.

2. Benzothiopyranopyridinones telles que définies par la formule (I) de la revendication 1, ainsi que leurs sels, caractérisées en ce que dans ladite formule (I), A représente un atome d'hydrogène et B

représente un atome d'hydrogène, un radical méthyle, méthoxy, un radical nitro ou acétamido ou un atome de chlore ou de fluor ou B forme avec A et les atomes de carbone auxquels ils sont liés un cycle benzénique, C représente un atome d'hydrogène ou un radical méthoxy et D représente un atome d'hydrogène ou forme avec C et les atomes de carbone auxquels ils sont liés un cycle benzénique, R a la signification déjà indiquée.

3. Benzothiopyranopyridinones telles que définies à la revendication 1 ou 2, ainsi que leurs sels, caractérisées en ce que R représente un atome d'hydrogène ou un radical R′ dans lequel R′ représente un radical alcoyle, linéaire ou ramifié, renfermant de 1 à 9 atomes de carbone, un radical benzyle, un radical cyclohexylméthyle ou un groupement p-fluorophényl-4-oxo butyl ou éthoxy carbonyléthyle.

4. Benzothiopyranopyridinones telles que définies à l'une quelconque des revendications 1, 2 ou 3, ainsi que leurs sels, caractérisées en ce que A, B, C et D représentent un atome d'hydrogène.

5. La 1,2,3,4-tétrahydro 2-cyclohexylméthyl 10H-/1/-benzothiopyrano /3,2-c/ pyridin-10-one et ses sels.

6. Procédé de préparation des nouvelles benzothiopyranopyridinones telles que définies par la formule (I) de la revendication 1, ainsi que de leurs sels, caractérisé en ce que l'on condense le thiol de formule (II) :

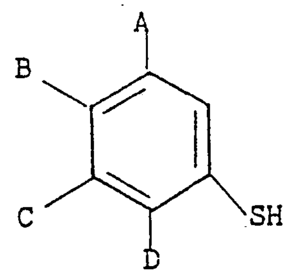

(II)

dans laquelle A, B, C et D ont la signification indiquée à la revendication 1 avec une 4-pipéridone ou un sel de 4-pipéridone de formule

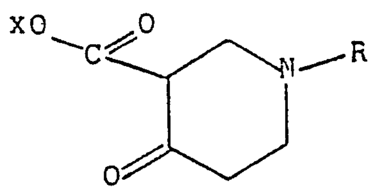

(III)

dans laquelle X représente un radical alcoyle renfermant de 1 à 8 atomes de carbone et R est défini comme à la revendication 1 pour obtenir un produit de formule

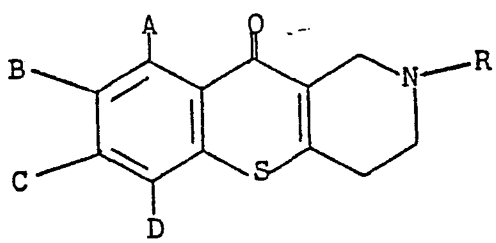

(I)

dans laquelle A, B, C, D et R ont la signification déjà indiquée le cas échéant, isole et, si désiré, salifie ledit produit de formule (I) ainsi obtenu, ou, à condition d'avoir préparé un produit de formule (I) dans laquelle R représente un atome d'hydrogène,

soit on fait réagir ce dernier avec un dérivé halogéné de formule

Hal—R′ (IV)

dans laquelle Hal représente un atome de chlore, de brome ou d'iode et R′ a la signification indiquée à la revendication 1, à l'exception d'un radical phényle, pour obtenir un produit de formule :

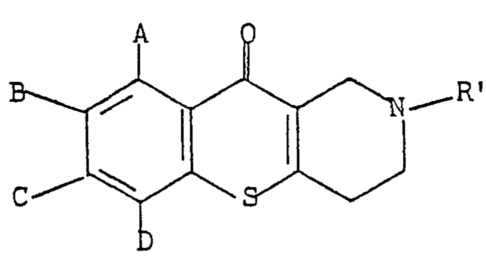

(I′)

dans laquelle A, B, C, E et R' ont la signification déjà indiquée et le cas échéant, isole et, si désiré, salifie ledit produit de formule (I') ainsi obtenu,

soit on fait réagir un produit de formule (I) dans laquelle R représente un atome d'hydrogène, avec un produit de formule

$$CH_2 = CH—Z' \quad (V)$$

dans laquelle Z' représente un radical alcoxycarbonyl, cyano ou carbamoyl, pour obtenir un produit de formule

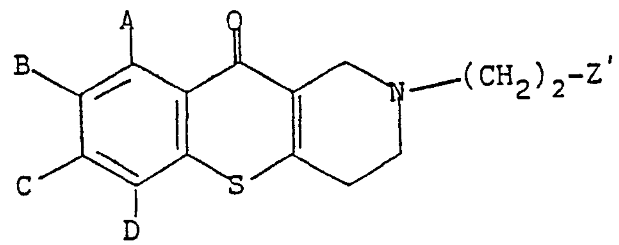

(I")

dans laquelle A, B, C, D, et Z' ont la signification déjà indiquée, isole et, si désiré, salifie ledit produit de formule (I") ainsi obtenu.

7. Procédé selon la revendication 6, caractérisé en ce que :

la condensation du thiol de formule (II) avec la 4-pipéridone de formule (III) est effectuée par chauffage pendant plusieurs heures à plusieurs jours, au sein d'un acide fort tel que l'acide polyphosphorique ;

lorsque l'on utilise un sel de 4-pipéridone, il s'agit du chlorhydrate ;

la réaction du produit de formule (I) dans laquelle R représente un atome d'hydrogène avec le dérivé halogéné de formule (IV) est effectuée au sein d'un alcanol tel que l'éthanol, au reflux du mélange, en présence d'une base aminée telle que la triéthylamine ;

la réaction du produit de formule (I) avec le produit de formule (V) est effectuée au sein d'un alcanol tel que l'éthanol, au reflux du mélange.

8. Variante du procédé selon la revendication 6, caractérisée en ce que la réaction du produit de formule (I) dans laquelle R représente un atome d'hydrogène avec le dérivé halogéné de formule (IV) est effectuée à chaud au sein d'une base aminée telle que la diméthylformamide et en présence d'iodure de potassium et d'un carbonate alcalin tel que le carbonate de potassium.

9. Médicaments caractérisés en ce qu'ils sont constitués par les nouvelles benzothiopyranopyridinones telles que définies par la formule (I) de la revendication 1, ainsi que par leurs sels pharmaceutiquement acceptables.

10. Médicaments caractérisés en ce qu'ils sont constitués par les nouveaux dérivés tels que définis à l'une des revendications 2, 3 ou 4, ainsi que par leurs sels pharmaceutiquement acceptables.

11. Médicaments caractérisés en ce qu'ils sont constitués par le dérivé tel que défini à la revendication 5, ainsi que par ses sels pharmaceutiquement acceptables.

12. Compositions pharmaceutiques, caractérisées en ce qu'elles renferment à titre de principe actif, l'un au moins des médicaments tels que définis à l'une quelconque des revendications 9, 10 ou 11.

**Revendications** (pour l'Etat contractant AT)

1. Procédé pour préparer des benzothiopyranopyridinones et leurs sels, répondant à la formule :

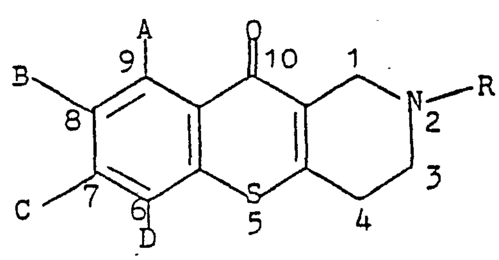

(I)

dans laquelle A, B, C et D, identiques ou différents, représentent un atome d'hydrogène, un radical alcoyle linéaire ou ramifié renfermant de 1 à 6 atomes de carbone, un radical alcoxy linéaire ou ramifié renfermant de 1 à 6 atomes de carbone, un radical nitro, un radical amino, un radical acylamino renfermant de 2 à 7 atomes de carbone ou un atome d'halogène, ou l'un des groupes A et B ou C et D

représente avec les atomes de carbone auxquels ils sont liés, un cycle benzénique et l'autre est défini comme ci-dessus,

R représente un atome d'hydrogène ou un radical R' dans lequel R' représente un radical alcoyle, linéaire ou ramifié, renfermant de 1 à 9 atomes de carbone ou alcényle, linéaire ou ramifié, renfermant de 2 à 9 atomes de carbone, éventuellement substitué par un radical hydroxy, un radical cycloalcoyle renfermant de 3 à 9 atomes de carbone, un radical phényle, un radical aralcoyle renfermant de 7 à 9 atomes de carbone ou un groupement $-(CH_2)_n-Z$ dans lequel n est un nombre entier qui varie de 1 à 4 et Z représente un radical cycloalcoyle ou cycloalcényle renfermant de 3 à 9 atomes de carbone, un radical dioxanyl, un radical 2-oxo benzimidazolyl, un radical benzoyl éventuellement substitué par un atome d'halogène, un radical alcoxycarbonyl renfermant de 2 à 7 atomes de carbone, un radical cyano ou un radical cabamoyl, caractérisé en ce que l'on condense le thiol de formule (II):

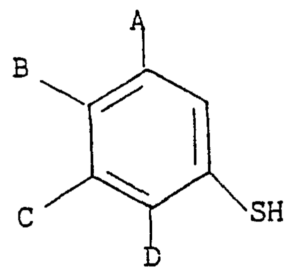

(II)

dans laquelle A, B, C et D ont la signification déjà indiquée avec une 4-pipéridone ou un sel de 4-pipéridone de formule

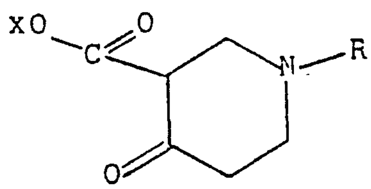

(III)

dans laquelle X représente un radical alcoyle renfermant de 1 à 8 atomes de carbone et R est défini comme précédemment pour obtenir un produit de formule

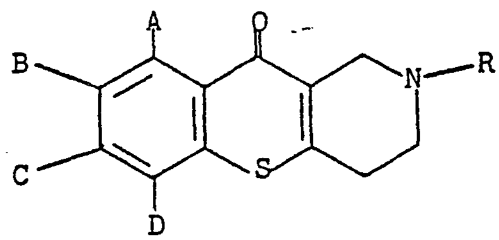

(I)

dans laquelle A, B, C, D et R ont la signification déjà indiquée le cas échéant, isole et, si désiré, salifie ledit produit de formule (I) ainsi obtenu, ou, à condition d'avoir préparé un produit de formule (I) dans laquelle R représente un atome d'hydrogène,

soit on fait réagir ce dernier avec un dérivé halogéné de formule

$$Hal—R' \quad (IV)$$

dans laquelle Hal représente un atome de chlore, de brome ou d'iode et R' a la signification indiquée précédemment à l'exception d'un radical phényle, pour obtenir un produit de formule :

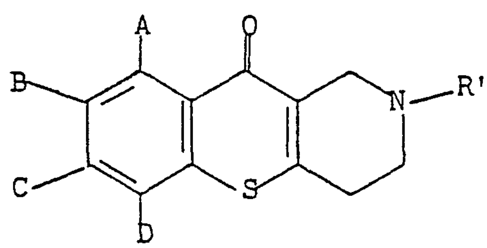

(I')

dans laquelle A, B, C, E et R' ont la signification déjà indiquée et le cas échéant, isole et, si désiré, salifie ledit produit de formule (I') ainsi obtenu,
soit on fait réagir un produit de formule (I) dans laquelle R représente un atome d'hydrogène, avec un produit de formule

$$CH_2 = CH—Z' \quad (V)$$

dans laquelle Z' représente un radical alcoxycarbonyl, cyano ou carbamoyl, pour obtenir un produit de formule

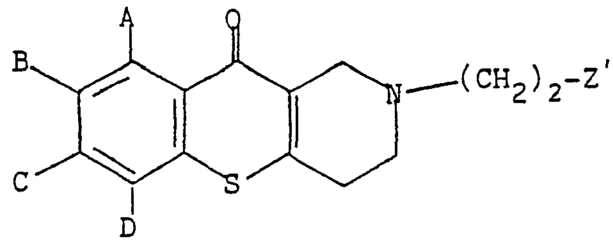

(I")

dans laquelle A, B, C, D et Z' ont la signification déjà indiquée, isole et, si désiré, salifie ledit produit de formule (I") ainsi obtenu.

2. Procédé selon la revendication 1, caractérisé en ce que :

la condensation du thiol de formule (II) avec la 4-pipéridone de formule (III) est effectuée par chauffage pendant plusieurs heures à plusieurs jours, au sein d'un acide fort tel que l'acide polyphosphorique ;

lorsque l'on utilise un sel de 4-pipéridone, il s'agit du chlorhydrate ;

la réaction du produit de formule (I) dans laquelle R représente un atome d'hydrogène avec le dérivé halogéné de formule (IV) est effectuée au sein d'un alcanol tel que l'éthanol, au reflux du mélange, en présence d'une base aminée telle que la triéthylamine ;

la réaction du produit de formule (I) avec le produit de formule (V) est effectuée au sein d'un alcanol tel que l'éthanol, au reflux du mélange.

3. Procédé selon la revendication 1, caractérisé en ce que la réaction du produit de formule (I) dans laquelle R représente un atome d'hydrogène avec le dérivé halogéné de formule (IV) est effectuée à chaud au sein d'une base aminée telle que la diméthylformamide et en présence d'iodure de potassium et d'un carbonate alcalin tel que le carbonate de potassium.

4. Procédé selon l'une quelconque des revendications 1 à 3, caractérisé en ce que l'on utilise au départ un thiol de formule (II), dans laquelle A représente un atome d'hydrogène et B représente un atome d'hydrogène, un radical méthyle, méthoxy, un radical nitro ou acétamido ou un atome de chlore ou de fluor ou B forme avec A et les atomes de carbone auxquels ils sont liés un cycle benzénique, C représente un atome d'hydrogène ou un radical méthoxy et D représente un atome d'hydrogène ou forme avec C et les atomes de carbone auxquels ils sont liés un cycle benzénique.

5. Procédé selon l'une quelconque des revendications 1 à 4, caractérisé en ce que l'on utilise au départ un dérivé de formule (IV) dans laquelle R' représente un radical alcoyle, linéaire ou ramifié, renfermant de 1 à 9 atomes de carbone, un radical benzyle, un radical cyclohexylméthyle ou un groupement p-fluorophényl-4-oxobutyl ou éthoxycarbonyléthyle.

6. Procédé selon l'une quelconque des revendications 1 à 5, caractérisé en ce que l'on utilise au départ un thiol de formule (II), dans laquelle A, B, C et D représentent un atome d'hydrogène.

7. Procédé selon la revendication 6, caractérisé en ce que l'on utilise au départ un dérivé de formule (IV) dans laquelle R' représente un radical cyclohexylméthyl.

**Claims** (for the Contracting States : BE, CH, DE, FR, IT, LI, LU, NL, SE)

1. Benzothiopyranopyridinones and their salts, characterized in that they respond to the formula :

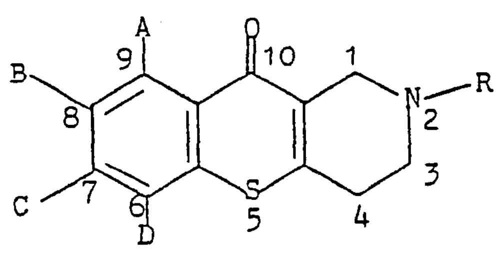

(I)

in which A, B, C and D, identical or different, represent a hydrogen atom, a linear or branched alkyl radical containing from 1 to 6 carbon atoms, a linear or branched alkoxy radical containing from 1 to 6 carbon atoms, a nitro radical, an amino radical, an acylamino radical containing from 2 to 7 carbon atoms or a halogen atom, or one of the groups A and B or C and D represents, together with the carbon atoms to which they are bonded, a benzene ring and the other is defined as above,

R represents a hydrogen atom or a radical R′ in which R′ represents a linear or branched alkyl radical, containing from 1 to 9 carbon atoms or a linear or branched alkenyl radical containing from 2 to 9 carbon atoms, possibly substituted by a hydroxy radical, a cycloalkyl radical containing from 3 to 9 carbon atoms, a phenyl radical, an aralkyl radical containing from 7 to 9 carbon atoms or a group $-(CH_2)_n-Z$ in which n is an integer varying from 1 to 4 and Z represents a cycloalkyl or cycloalkenyl radical containing from 3 to 9 carbon atoms, a dioxanyl radical, a 2-oxo-benzimidazolyl radical, a benzoyl radical possibly substituted by a halogen atom, an alkoxycarbonyl radical containing from 2 to 7 carbon atoms, a cyano radical or a carbamoyl radical.

2. Benzothiopyranopyridinones as defined by formula (I) of Claim 1, as well as their salts, characterized in that in the said formula (I), A represents a hydrogen atom and B represents a hydrogen atom, a methyl or methoxy radical, a nitro or acetamido radical, or a chlorine or fluorine atom, or B forms with A and the carbon atoms to which they are bonded a benzene ring, C represents a hydrogen atom or a methoxy radical and D represents a hydrogen atom or forms with C and the carbon atoms to which they are bonded a benzene ring, R has the significance already indicated.

3. Benzothiopyranopyridinones as defined in Claim 1 or 2, as well as their salts, characterized in that R represents a hydrogen atom or a radical R′ in which R′ represents a linear or branched alkyl radical containing from 1 to 9 carbon atoms, a benzyl radical, a cyclohexylmethyl radical or a p-fluorophenyl-4-oxo-butyl or an ethoxy-carbonylethyl group.

4. Benzothiopyranopyridinones as defined in any one of the Claims 1, 2 or 3, as well as their salts, characterized in that A, B, C and D represent a hydrogen atom.

5. 1,2,3,4-tetrahydro-2-cyclohexylmethyl-10H-[1]-benzothiopyrano-[3,2-c]pyridin-10-one and its salts.

6. Preparation process for the new benzothiopyranopyridinones as defined by formula (I) of Claim 1, as well as their salts, characterized in that the thiol of formula (II) :

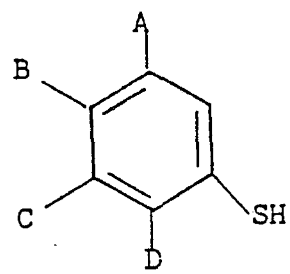

(II)

in which A, B, C and D have the significance indicated in Claim 1 is condensed with a 4-piperidone or a salt of 4-piperidone with the formula

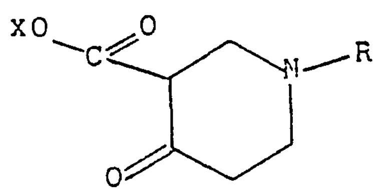

(III)

in which X represents an alkyl radical containing from 1 to 8 carbon atoms and R is defined as in Claim 1, in order to obtain a product with the formula

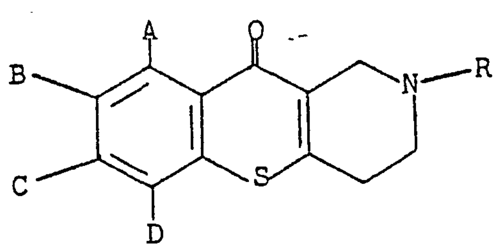

(I)

in which A, B, C, D and R have the significance already indicated, if necessary the said product so obtained with the formula (I) is isolated and if desired salified, or if a product with the formula (I) has been prepared in which R represents a hydrogen atom,
either this latter is made to react with a halogenated derivative with the formula

Hal—R' (IV)

in which Hal represents a chlorine, bromine or iodine atom and R' has the significance indicated in Claim 1 with the exception of a phenyl radical, in order to obtain a product with the formula :

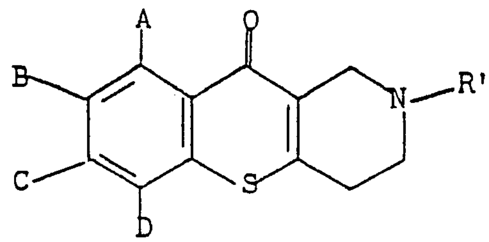

(I')

in which A, B, C, D and R' have the significance already indicated and if necessary, the said product so obtained with the formula (I') is isolated and if desired salified,
or a product with the formula (I) in which R represents a hydrogen atom is made to react with a product with the formula

$CH_2 = CH—Z'$ (V)

in which Z' represents an alkoxycarbonyl, cyano or carbamoyl radical, in order to obtain a product with the formula

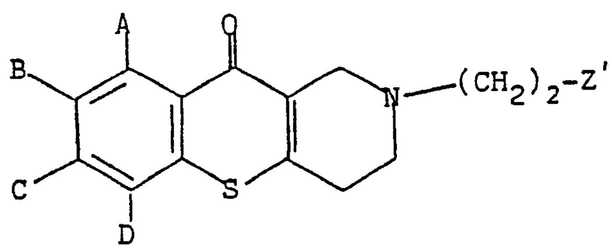

(I'')

in which A, B, C, D and Z' have the significance already indicated, and the said product so obtained with the formula (I'') is isolated and if desired salified.

7. Process according to Claim 6, characterized in that :

the condensation of the thiol with the formula (II) with the 4-piperidone with the formula (III) is carried out by heating during several hours to several days, in a strong acid such a polyphosphoric acid ;

when a salt of 4-piperidone is used, it would be the hydrochloride.

the reaction of the product with the formula (I) in which R represents a hydrogen atom with the halogenated derivative with the formula (IV) is carried out in an alkanol such as ethanol, at reflux of the mixture, in the presence of an amine base such as triethylamine ;

the reaction of the product with the formula (I) with the product with the formula (V) is carried out in an alkanol such as ethanol, at reflux of the mixture.

8. Variant of the process according to Claim 6, characterized in that the reaction of the product with the formula (I) in which R represents a hydrogen atom with the halogenated derivative with the formula (IV) is carried out hot in an amine base such as dimethylformamide and in the presence of potassium iodide and of an alkaline carbonate such as potassium carbonate.

9. Medicaments characterized in that they are constituted by the new benzothiopyranopyridinones as defined by the formula (I) of Claim 1, as well as by their pharmaceutically acceptable salts.

10. Medicaments characterized in that they are constituted by the new derivatives as defined in one of the Claims 2, 3 or 4, as well as by their pharmaceutically acceptable salts.

11. Medicaments characterized in that they are constituted by the derivative as defined in Claim 5, as well as by its pharmaceutically acceptable salts.

12. Pharmaceutical compositions, characterized in that they contain as active principle, one at least of the medicaments as defined in any one of the Claims 9, 10 or 11.

**Claims** (for the Contracting State AT)

1. Preparation process for benzothiopyranopyridinones and their salts, answering to the formula :

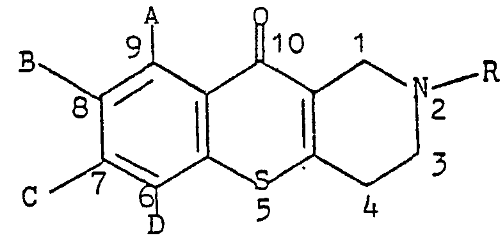

(I)

in which A, B, C and D, identical or different, represent a hydrogen atom, a linear or branched alkyl radical containing from 1 to 6 carbon atoms, a linear or branched alkoxy radical containing from 1 to 6 carbon atoms, a nitro radical, an amino radical, an acylamino radical containing from 2 to 7 carbon atoms or a halogen atom, or one of the groups A and B or C and D represents, with the carbon atoms to which they are bonded, a benzene ring and the other is defined as above,

R represents a hydrogen atom or a radical R′ in which R′ represents a linear or branched alkyl radical containing from 1 to 9 carbon atoms or a linear or branched alkenyl radical, containing from 2 to 9 carbon atoms, possibly substituted by a hydroxy radical, a cycloalkyl radical containing from 3 to 9 carbon atoms, a phenyl radical, an aralkyl radical containing from 7 to 9 carbon atoms or a group $-(CH_2)_n-Z$ in which n is an integer which varies from 1 to 4 and Z represents a cycloalkyl or cycloalkenyl radical containing from 3 to 9 carbon atoms, a dioxanyl radical, a 2-oxo-benzimidazolyl radical, a benzoyl radical possibly substituted by a halogen atom, an alkoxycarbonyl radical containing from 2 to 7 carbon atoms, a cyano radical or a carbamoyl radical, characterized in that the thiol with the formula (II) :

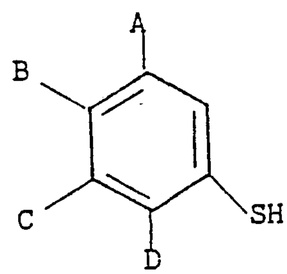

(II)

in which A, B, C and D have the significance already indicated is condensed with a 4-piperidone or a salt of 4-piperidone with the formula

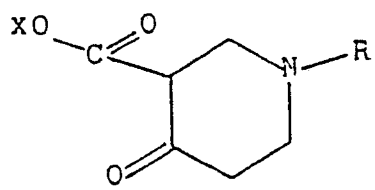

(III)

in which X represents an alkyl radical containing from 1 to 8 carbon atoms and R is defined as previously, in order to obtain a product with the formula

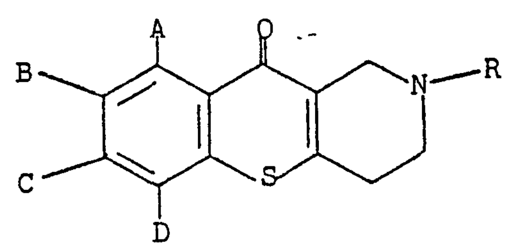

(I)

in which A, B, C, D and R have the significance already indicated, if necessary the said product so obtained with the formula (I) is isolated and if desired salified, or, if a product with the formula (I) in which R represents a hydrogen atom has been prepared, either this latter is made to react with a halogenated derivative with the formula

Hal—R' (IV)

in which Hal represents a chlorine, bromine or iodine atom and R' has the previously indicated significance, with the exception of a phenyl radical, in order to obtain a product with the formula :

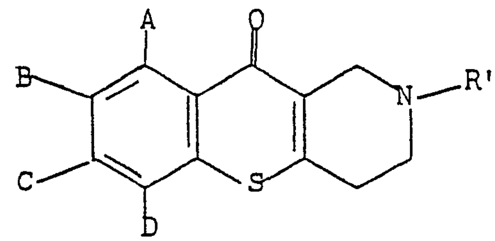

(I')

in which A, B, C, D and R' have the significance already indicated, and, if required, the said product so obtained with the formula (I') is isolated and if desired, salified,
or a product with the formula (I) in which R represents a hydrogen atom is made to react with a product with the formula

$$CH_2 = CH—Z' \quad (V)$$

in which Z' represents an alkoxycarbonyl, cyano or carbamoyl radical, in order to obtain a product with the formula

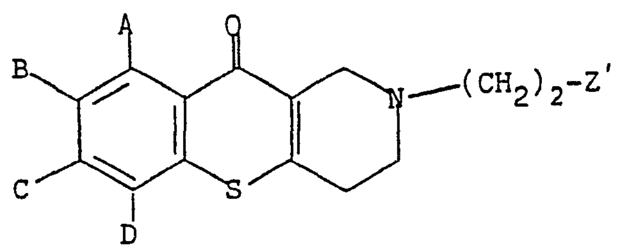

(I'')

in which A, B, C, D and Z' have the significance already indicated, and the said product so obtained with the formula (I'') is isolated and if desired salified.

2. Process according to Claim 1, characterized in that :

the condensation of the thiol with the formula (II) with the 4-piperidone with the formula (III) is carried out by heating for several hours to several days in a strong acid such as polyphosphoric acid ;

when a salt of 4-piperidone is used, it would be the hydrochloride ;

the reaction of the product with the formula (I) in which R represents a hydrogen atom with the halogenated derivative with the formula (IV) is carried out in an alkanol such as ethanol, at reflux of the mixture, in the presence of an amine base such as triethylamine ;

the reaction of the product with the formula (I) with the product with the formula (V) is carried out in an alkanol such as ethanol at reflux of the mixture.

3. Process according to Claim 1, characterized in that the reaction of the product with the formula (I) in which R represents a hydrogen atom with the halogenated derivative with the formula (IV) is carried out hot in an amine base such as dimethylformamide and in the presence of potassium iodide and of an alkaline carbonate such as potassium carbonate.

4. Process according to any one of the Claims 1 to 3, characterized in that at the start a thiol with the formula (II) is used, in which A represents a hydrogen atom and B represents a hydrogen atom, a methyl or methoxy radical, a nitro or acetamido radical or a chlorine or fluorine atom, or B forms with A and the carbon atoms to which they are bonded a benzene ring, C represents a hydrogen atom or a methoxy radical and D represents a hydrogen atom, or forms with C and the carbon atoms to which they are bonded a benzene ring.

5. Process according to any one of the Claims 1 to 4, characterized in that at the start a derivative with the formula (IV) is used in which R' represents a linear or branched alkyl radical, containing from 1 to 9 carbon atoms, a benzyl radical, a cyclohexylmethyl radical or a p-fluorophenyl-4-oxobutyl or ethoxycarbonylethyl group.

6. Process according to any one of the Claims 1 to 5, characterized in that at the start a thiol with the formula (II) is used in which A, B, C and D represent a hydrogen atom.

7. Process according to Claim 6, characterized in that at the start a derivative with the formula (IV) is used in which R' represents a cyclohexylmethyl radical.

**Patentansprüche** (für die Vertragsstaaten : BE, CH, DE, FR, IT, LI, LU, NL, SE)

1. Benzothiopyranopyridinone und deren Salze, dadurch gekennzeichnet, daß sie der Formel

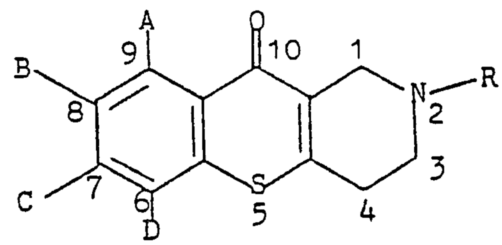

(I)

entsprechen, worin A, B, C und D, die gleich oder voneinander verschieden sind, ein Wasserstoffatom, einen linearen oder verzweigten Alkylrest mit 1 bis 6 Kohlenstoffatomen, eine lineare oder verzweigte Alkoxygruppe mit 1 bis 6 Kohlenstoffatomen, eine Nitrogruppe, eine Aminogruppe, einen Acylaminorest mit 2 bis 7 Kohlenstoffatomen oder ein Halogenatom bedeuten oder eine der Gruppen A und B oder C und D mit den Kohlenstoffatomen, an die sie gebunden sind, einen Benzolring bildet und die andere wie vorstehend definiert ist,

R ein Wasserstoffatom oder einen Rest R' bedeutet, worin R' einen linearen oder verzweigten Alkylrest mit 1 bis 9 Kohlenstoffatomen oder einen linearen oder verzweigten Alkenylrest mit 2 bis 9 Kohlenstoffatomen, gegebenenfalls substituiert durch eine Hydroxygruppe, einen Cycloalkylrest mit 3 bis 9 Kohlenstoffatomen, einen Phenylrest, einen Aralkylrest mit 7 bis 9 Kohlenstoffatomen oder eine Gruppe $-(CH_2)_n-Z$ bedeutet, worin n eine ganze Zahl von 1 bis 4 bedeutet und Z einen Cycloalkyl- oder Cycloalkenylrest mit 3 bis 9 Kohlenstoffatomen, einen Dioxanylrest, eine 2-Oxobenzimidazolylgruppe, eine gegebenenfalls durch ein Halogenatom substituierte Benzoylgruppe, einen Alkoxycarbonylrest mit 2 bis 7 Kohlenstoffatomen, einen Cyanorest oder eine Carbamoylgruppe bedeutet.

2. Benzothiopyranopyridinone der Formel (I) gemäß Anspruch 1 sowie deren Salze, dadurch gekennzeichnet, daß in der Formel (I) A ein Wasserstoffatom bedeutet und B ein Wasserstoffatom, einen Methyl- oder Methoxyrest, eine Nitro- oder Acetamidogruppe oder ein Chlor- oder Fluoratom darstellt oder B mit A und den Kohlenstoffatomen, an die sie gebunden sind, einen Benzolring bildet, C ein Wasserstoffatom oder eine Methoxygruppe bedeutet und D ein Wasserstoffatom darstellt oder mit C und den Kohlenstoffatomen, an die sie gebunden sind, einen Benzolring bildet und R die angegebene Bedeutung besitzt.

3. Benzothiopyranopyridinone gamäß Anspruch 1 oder 2 sowie deren Salze, dadurch gekennzeichnet, daß R ein Wasserstoffatom oder einen Rest R' darstellt, worin R' einen linearen oder verzweigten Alkylrest mit 1 bis 9 Kohlenstoffatomen, einen Benzylrest, einen Cyclohexylmethylrest oder eine p-Fluorphenyl-4-oxobutyl- oder Ethoxycarbonylethylgruppe bedeutet.

4. Benzothiopyranopyridinone gemäß einem der Ansprüche 1, 2 oder 3 sowie deren Salze, dadurch gekennzeichnet, daß A, B, C und D ein Wasserstoffatom bedeuten.

5. 1,2,3,4-Tetrahydro-2-cyclohexylmethyl-10H-[1]-benzothiopyrano [3,2-c] pyridin-10-on und dessen Salze.

6. Verfahren zur Herstellung der neuen Benzothiopyranopyridinone der Formel (I) gemäß Anspruch 1 sowie von deren Salzen, dadurch gekennzeichnet, daß man das Thiol der Formel (II)

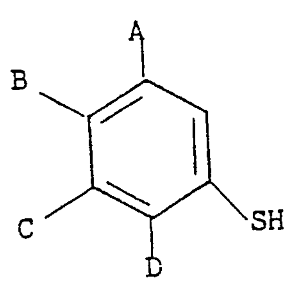

(II)

worin A, B, C und D die Anspruch 1 angegebene Bedeutung besitzen, mit einem 4-Piperidon oder einem 4-Piperidonsalz der Formel

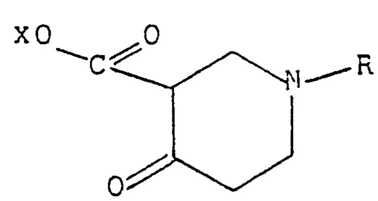

(III)

worin X einen Alkylrest mit 1 Bis 8 Kohlenstoffatomen darstellt und R wie in Anspruch 1 definiert ist, kondensiert, um ein Produkt der Formel

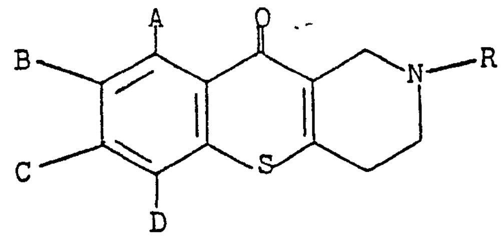

(I)

zu erhalten, worin A, B, C, D und R die angegebene Bedeutung besitzen, gegebenenfalls das so erhaltene Produkt der Formel (I) isoliert und gewünschtenfalls in ein Salz überführt, oder wenn man ein Produkt der Formel (I) erhalten hat, worin R ein Wasserstoffatom bedeutet,
entweder dieses letztere mit einem Halogenderivat der Formel

HAL—R' (IV)

worin Hal ein Chlor-, Brom- oder Jodatom bedeutet und R' die in Anspruch 1 angegebene Bedeutung besitzt, mit Ausnahme eines Phenylrestes, umsetzt, um ein Produkt der Formel

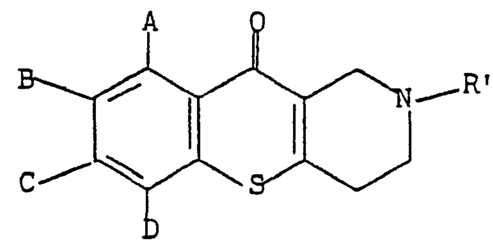

(I')

zu erhalten, worin A, B, C, D und R' die angegebene Bedeutung besitzen, und gegebenenfalls das so erhaltene Produkt der Formel (I') isoliert und gewünschtenfalls in ein Salz überführt,
oder ein Produkt der Formel (I), worin R ein Wasserstoffatom bedeutet, mit einem Produkt der Formel

$CH_2 = CH—Z'$ (V)

umsetzt, worin Z' eine Alkoxycarbonyl-, Cyano- oder Carbamoylgruppe bedeutet, um ein Produkt der Formel

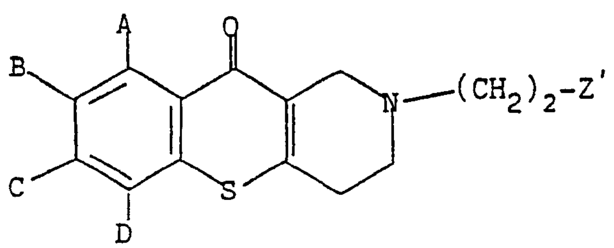

(I'')

zu erhalten, worin A, B, C, D und Z' die angegebene Bedeutung besitzen, das so erhaltene Produkt der Formel (I'') isoliert und gewünschtenfalls in ein Salz überführt.

7. Verfahren gemäß Anspruch 6, dadurch gekennzeichnet, daß

die Kondensation des Thiols der Formel (II) mit dem 4-Piperidon der Formel (III) durch Erhitzen während mehrerer Stunden bis zu mehreren Tagen in dem Medium einer starken Säure, wie Polyphosphorsäure, durchgeführt wird ;

bei Verwendung eines 4-Piperidonsalzes es sich um das Hydrochlorid handelt ;

Die Umsetzung des Produkts der Formel (I), worin R ein Wasserstoffatom bedeutet, mit dem Halogenderivat der Formel (IV) in dem Medium eines Alkanols, wie Ethanol, unter Rückfluß des Gemisches in Anwesenheit einer Stickstoffbase, wie Triethylamin, durchgeführt wird ;

die Umsetzung des Produkts der Formel (I) mit dem Produkt der Formel (V) in dem Medium eines Alkanols, wie Ethanol, unter Rückfluß des Gemisches durchgeführt wird.

8. Abänderung des Verfahrens gemäß Anspruch 6, dadurch gekennzeichnet, daß die Umsetzung des Produkts der Formel (I), worin R ein Wasserstoffatom bedeutet, mit dem Halogenderivat der Formel (IV) in

der Wärme in dem Medium einer Stickstoffbase, wie Dimethylformamid, und in Anwesenheit von Kaliumjodid und eines Alkalicarbonats, wie Kaliumcarbonat, durchgeführt wird.

9. Arzneimittel, dadurch gekennzeichnet, daß sie aus den neuen Benzothiopyranopyridinonen der Formel (I) gemäß Anspruch 1 sowie aus deren pharmazeutisch verträglichen Salzen bestehen.

10. Arzneimittel, dadurch gekennzeichnet, daß sie aus den neuen Derivaten gemäß einem der Ansprüche 2, 3 oder 4 sowie aus deren pharmazeutisch verträglichen Salzen bestehen.

11. Arzneimittel, dadurch gekennzeichnet, daß sie aus dem Derivat gemäß Anspruch 5 sowie aus dessen pharmazeutisch verträglichen Salzen bestehen.

12. Pharmazeutische Zusammensetzungen, dadurch gekennzeichnet, daß sie als Wirkstoff zumindest eines der Arzneimittel gemäß einem der Ansprüche 9, 10 oder 11 enthalten.

**Patentansprüche** (für den Vertragsstaat AT)

1. Verfahren zur Herstellung von Benzothiopyranopyridinonen und deren Salzen der Formel

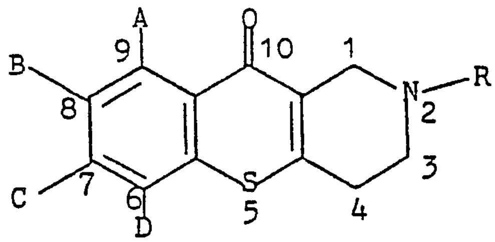

(I)

worin A, B, C und D, die gleich oder voneinander verschieden sind, ein Wasserstoffatom, einen linearen oder verzweigten Alkylrest mit 1 bis 6 Kohlenstoffatomen, eine lineare oder verzweigte Alkoxygruppe mit 1 bis 6 Kohlenstoffatomen, eine Nitrogruppe, eine Aminogruppe, einen Acylaminorest mit 2 bis 7 Kohlenstoffatomen oder ein Halogenatom bedeuten oder eine der Gruppen A und B oder C und D mit den Kohlenstoffatomen, an die sie gebunden sind, einen Benzolring bildet und die andere wie vorstehend definiert ist,

R ein Wasserstoffatom oder einen Rest R' bedeutet, worin R' einen linearen oder verzweigten Alkylrest mit 1 bis 9 Kohlenstoffatomen oder einen linearen oder verzweigten Alkenylrest mit 2 bis 9 Kohlenstoffatomen, gegebenenfalls substituiert durch eine Hydroxygruppe, einen Cycloalkylrest mit 3 bis 9 Kohlenstoffatomen, einen Phenylrest, einen Aralkylrest mit 7 bis 9 Kohlenstoffatomen oder eine Gruppe $-(CH_2)_n-Z$ bedeutet, worin n eine ganze Zahl von 1 bis 4 bedeutet und Z einen Cycloalkyl- oder Cycloalkenylrest mit 3 bis 9 Kohlenstoffatomen, einen Dioxanylrest, eine 2-Oxobenzimidazolylgruppe, eine gegebenenfalls durch ein Halogenatom substituierte Benzoylgruppe, einen Alkoxycarbonylrest mit 2 bis 7 Kohlenstoffatomen, einen Cyanorest oder eine Carbamoylgruppe bedeutet, dadurch gekennzeichnet, daß man das Thiol der Formel (II)

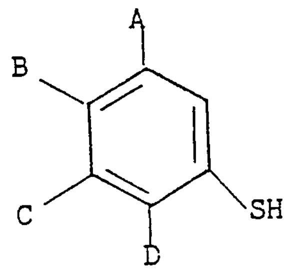

(II)

worin A, B, C und D die angegebene Bedeutung besitzen, mit einem 4-Piperidon oder einem 4-Piperidonsalz der Formel

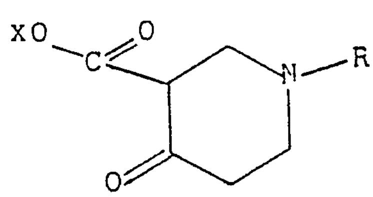

(III)

worin X einen Alkylrest mit 1 bis 8 Kohlenstoffatomen bedeutet und R wie vorstehend definiert ist, kondensiert, um ein Produkt der Formel

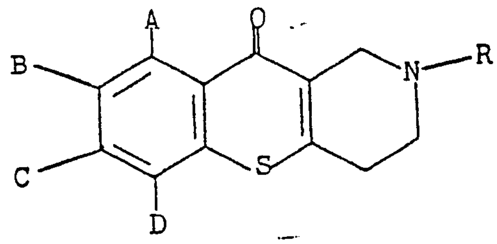

(I)

zu erhalten, worin A, B, C, D und R die angegebene Bedeutung besitzen, das so erhaltene Produkt der Formel (I) gegebenenfalls isoliert und gewünschtenfalls in ein Salz überführt, oder wenn man ein Produkt der Formel (I) hergestellt hat, worin R ein Wasserstoffatom bedeutet,
entweder dieses letztere mit einem Halogenderivat der Formel

$$Hal—R' \quad (IV)$$

worin Hal ein Chlor-, Brom- oder Jodatom bedeutet und R' die vorstehend angegebene Bedeutung mit Ausnahme eines Phenylrestes besitzt, umsetzt, um ein Produkt der Formel

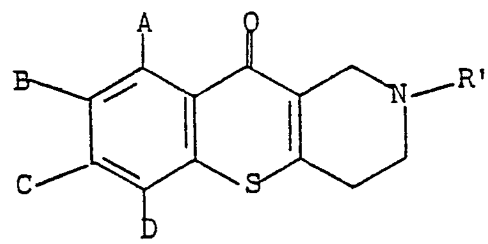

(I')

zu erhalten, worin A, B, C, D und R' die angegebene Bedeutung besitzen, und das so erhaltene Produkt der Formel (I') gegebenenfalls isoliert und gewünschtenfalls in ein Salz überführt,
oder ein Produkt der Formel (I), worin R ein Wasserstoffatom bedeutet, mit einem Produkt der Formel

$$CH_2 = CH—Z' \quad (V)$$

umsetzt, worin Z' einen Alkoxycarbonyl-, Cyano- oder Carbamoylrest bedeutet, um ein Produkt der Formel

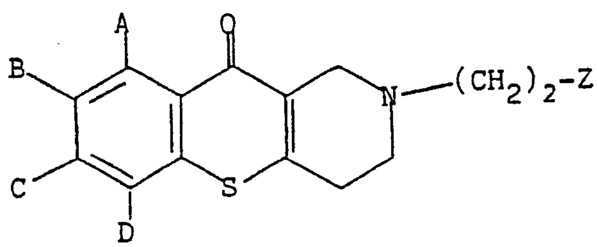

(I'')

zu erhalten, worin A, B, C, D und Z' die angegebene Bedeutung besitzen, das so erhaltene Produkt der Formel (I'') isoliert und gewünschtenfalls in ein Salz überführt.

2. Verfahren gemäß Anspruch 1, dadurch gekennzeichnet, daß
die Kondensation des Thiols der Formel (II) mit dem 4-Piperidon der Formel (III) durch Erhitzen während mehrerer Stunden bis mehreren Tagen in dem Medium einer starken Säure, wie Polyphosphorsäure, durchgeführt wird;
bei Verwendung eines 4-Piperidonsalzes es sich um das Hydrochlorid handelt;
die Umsetzung des Produkts der Formel (I), worin R ein Wasserstoffatom bedeutet, mit dem Halogenderivat der Formel (IV) in dem Medium eines Alkanols, wie Ethanol, unter Rückfluß des Gemisches in Anwesenheit einer Stickstoffbase, wie Triethylamin, durchgeführt wird;
die Umsetzung des Produkts der Formel (I) mit dem Produkt der Formel (V) in dem Medium eines Alkanols, wie Ethanol, unter Rückfluß des Gemisches durchgeführt wird.

3. Verfahren gemäß Anspruch 1, dadurch gekennzeichnet, daß die Umsetzung des Produkts der Formel (I), worin R ein Wasserstoffatom bedeutet, mit dem Halogen-derivat der Formel (IV) in der Wärme in dem Medium einer Stickstoffbase, wie Dimethylformamid, und in Anwesenheit von Kaliumjodid und einem Alkalicarbonat, wie Kaliumcarbonat, durchgeführt wird.

4. Verfahren gemäß einem der Ansprüche 1 bis 3, dadurch gekennzeichnet, daß man von einem Thiol der Formel (II) ausgeht, worin A ein Wasserstoffatom bedeutet und B ein Wasserstoffatom, einen

Methyl- oder Methoxyrest, eine Nitro- oder Acetamidogruppe oder ein Chlor- oder Fluoratom darstellt oder B mit A und den Kohlenstoffatomen, an die sie gebunden sind, einen Benzolring bildet, C ein Wasserstoffatom oder einen Methoxyrest bedeutet und D ein Wasserstoffatom darstellt oder mit C und den Kohlenstoffatomen, an die sie gebunden sind, einen Benzolring bildet.

5. Verfahren gemäß einem der Ansprüche 1 bis 4, dadurch gekennzeichnet, daß man von einem Derivat der Formel (IV) ausgeht, worin R' einen linearen oder verzweigten Alkylrest mit 1 bis 9 Kohlenstoffatomen, einen Benzylrest, einen Cyclohexylmethylrest oder eine p-Fluor-phenyl-4-oxobutyl- oder Ethoxycarbonylethylgruppe bedeutet.

6. Verfahren gemäß einem der Ansprüche 1 bis 5, dadurch gekennzeichnet, daß man von einem Thiol der Formel (II) ausgeht, worin A, B, C und D ein Wasserstoffatom bedeuten.

7. Verfahren gemäß Anspruch 6, dadurch gekennzeichnet, daß man von einem Derivat der Formel (IV) ausgeht, worin R' einen Cyclohexylmethylrest bedeutet.